# EUROPEAN PATENT APPLICATION

(11) **EP 2 383 334 A1**
(43) Date of publication of application: **02.11.2011**
(21) Application number: 10733577.0
(22) Date of filing: 22.01.2010
(51) Int. Cl.: C12N 5/10, A61L 27/00, C07K 14/035, C07K 14/435, C12N 15/09

(54) **FEEDER CELL FOR TARGET CELL INDUCTION**

(30) Priority: 23.01.2009 JP 2009013068
(71) Applicant: Osaka University, Suita-shi Osaka 565-0871 (JP)
(72) Inventor: INOUE, Tomoyuki, Suita-shi Osaka 565-0871 (JP); TAKAMATSU, Fumihiko, Suita-shi Osaka 565-0871 (JP); MAEDA, Naoyuki, Suita-shi Osaka 565-0871 (JP); NISHIDA, Kohji, Suita-shi Osaka 565-0871 (JP)
(74) Representative: Kalhammer, Georg
(86) International application number: PCT/JP2010/050846
(87) International publication number: WO 2010/084970

(57) **Abstract**

The present invention provides a feeder cell for target cell induction, wherein the feeder cell is transduced with an immortalizing gene and a suicide gene; and a production process for a target cell sheet using the feeder cell for target cell induction.

SEQUENCE LISTING

<110>  Osaka University

<120>  Feeder cell for induction of taget cell

<130>  O11F3297

<150>  JP 2009-013068
<151>  2009-01-23

<160>  14

<170>  PatentIn version 3.1

<210>  1
<211>  3399
<212>  DNA
<213>  Homo sapiens

<220>
<221>  CDS
<222>  (1)..(3396)
<223>

<400>  1
atg ccg cgc gct ccc cgc tgc cga gcc gtg cgc tcc ctg ctg cgc agc      48
cac tac cgc gag gtg ctg ccg ctg gcc acg ttc gtg cgg cgc ctg ggg      96
ccc cag ggc tgg cgg ctg gtg cag cgc ggg gac ccg gcg gct ttc cgc      144
gcg ctg gtg gcc cag tgc ctg gtg tgc gtg ccc tgg gac gca cgg ccg      192
ccc ccc gcc gcc ccc tcc ttc cgc cag gtg tcc tgc ctg aag gag ctg      240
gtg gcc cga gtg ctg cag agg ctg tgc gag cgc ggc gcg aag aac gtg      288
ctg gcc ttc ggc ttc gcg ctg ctg gac ggg gcc cgc ggg ggc ccc ccc      336
gag gcc ttc acc acc agc gtg cgc agc tac ctg ccc aac acg gtg acc      384
gac gca ctg cgg ggg agc ggg gcg tgg ggg ctg ctg ctg cgc cgc gtg      432
ggc gac gac gtg ctg gtt cac ctg ctg gca cgc tgc gcg ctc ttt gtg        480
ctg gtg gct ccc agc tgc gcc tac cag gtg tgc ggg ccg ccg ctg tac        528
cag ctc ggc gct gcc act cag gcc cgg ccc ccg cca cac gct agt gga        576
ccc cga agg cgt ctg gga tgc gaa cgg gcc tgg aac cat agc gtc agg        624
gag gcc ggg gtc ccc ctg ggc ctg cca gcc ccg ggt gcg agg agg cgc        672
ggg ggc agt gcc agc cga agt ctg ccg ttg ccc aag agg ccc agg cgt        720
ggc gct gcc cct gag ccg gag cgg acg ccc gtt ggg cag ggg tcc tgg        768
gcc cac ccg ggc agg acg cgt gga ccg agt gac cgt ggt ttc tgt gtg        816
gtg tca cct gcc aga ccc gcc gaa gaa gcc acc tct ttg gag ggt gcg        864
ctc tct ggc acg cgc cac tcc cac cca tcc gtg ggc cgc cag cac cac        912
gcg ggc ccc cca tcc aca tcg cgg cca cca cgt ccc tgg gac acg cct        960
tgt ccc ccg gtg tac gcc gag acc aag cac ttc ctc tac tcc tca ggc       1008
gac aag gag cag ctg cgg ccc tcc ttc cta ctc agc tct ctg agg ccc       1056
agc ctg act ggc gct cgg agg ctc gtg gag acc atc ttt ctg ggt tcc       1104
agg ccc tgg atg cca ggg act ccc cgc agg ttg ccc cgc ctg ccc cag       1152
cgc tac tgg caa atg cgg ccc ctg ttt ctg gag ctg ctt ggg aac cac     1200
gcg cag tgc ccc tac ggg gtg ctc ctc aag acg cac tgc ccg ctg cga     1248
gct gcg gtc acc cca gca gcc ggt gtc tgt gcc cgg gag aag ccc cag     1296
ggc tct gtg gcg gcc ccc gag gag gag gac aca gac ccc cgt cgc ctg     1344
gtg cag ctg ctc cgc cag cac agc agc ccc tgg cag gtg tac ggc ttc     1392
gtg cgg gcc tgc ctg cgc cgg ctg gtg ccc cca ggc ctc tgg ggc tcc     1440
agg cac aac gaa cgc cgc ttc ctc agg aac acc aag aag ttc atc tcc     1488
ctg ggg aag cat gcc aag ctc tcg ctg cag gag ctg acg tgg aag atg     1536
agc gtg cgg gac tgc gct tgg ctg cgc agg agc cca ggg gtt ggc tgt     1584
gtt ccg gcc gca gag cac cgt ctg cgt gag gag atc ctg gcc aag ttc     1632
ctg cac tgg ctg atg agt gtg tac gtc gtc gag ctg ctc agg tct ttc     1680
ttt tat gtc acg gag acc acg ttt caa aag aac agg ctc ttt ttc tac     1728
cgg aag agt gtc tgg agc aag ttg caa agc att gga atc aga cag cac     1776
ttg aag agg gtg cag ctg cgg gag ctg tcg gaa gca gag gtc agg cag     1824
cat cgg gaa gcc agg ccc gcc ctg ctg acg tcc aga ctc cgc ttc atc     1872
ccc aag cct gac ggg ctg cgg ccg att gtg aac atg gac tac gtc gtg     1920
gga gcc aga acg ttc cgc aga gaa aag agg gcc gag cgt ctc acc tcg      1968
agg gtg aag gca ctg ttc agc gtg ctc aac tac gag cgg gcg cgg cgc      2016
ccc ggc ctc ctg ggc gcc tct gtg ctg ggc ctg gac gat atc cac agg      2064
gcc tgg cgc acc ttc gtg ctg cgt gtg cgg gcc cag gac ccg ccg cct      2112
gag ctg tac ttt gtc aag gtg gat gtg acg ggc gcg tac gac acc atc      2160
ccc cag gac agg ctc acg gag gtc atc gcc agc atc atc aaa ccc cag      2208
aac acg tac tgc gtg cgt cgg tat gcc gtg gtc cag aag gcc gcc cat      2256
ggg cac gtc cgc aag gcc ttc aag agc cac gtc tct acc ttg aca gac      2304
ctc cag ccg tac atg cga cag ttc gtg gct cac ctg cag gag acc agc      2352
ccg ctg agg gat gcc gtc gtc atc gag cag agc tcc tcc ctg aat gag      2400
gcc agc agt ggc ctc ttc gac gtc ttc cta cgc ttc atg tgc cac cac      2448
gcc gtg cgc atc agg ggc aag tcc tac gtc cag tgc cag ggg atc ccg      2496
cag ggc tcc atc ctc tcc acg ctg ctc tgc agc ctg tgc tac ggc gac      2544
atg gag aac aag ctg ttt gcg ggg att cgg cgg gac ggg ctg ctc ctg      2592
cgt ttg gtg gat gat ttc ttg ttg gtg aca cct cac ctc acc cac gcg      2640
aaa acc ttc ctc agg acc ctg gtc cga ggt gtc cct gag tat ggc tgc      2688
gtg gtg aac ttg cgg aag aca gtg gtg aac ttc cct gta gaa gac gag      2736
gcc ctg ggt ggc acg gct ttt gtt cag atg ccg gcc cac ggc cta ttc      2784
ccc tgg tgc ggc ctg ctg ctg gat acc cgg acc ctg gag gtg cag agc      2832
gac tac tcc agc tat gcc cgg acc tcc atc aga gcc agt ctc acc ttc      2880
aac cgc ggc ttc aag gct ggg agg aac atg cgt cgc aaa ctc ttt ggg      2928
gtc ttg cgg ctg aag tgt cac agc ctg ttt ctg gat ttg cag gtg aac      2976
agc ctc cag acg gtg tgc acc aac  atc tac aag atc ctc  ctg ctg cag    3024
gcg tac  agg ttt cac gca tgt  gtg ctg cag ctc cca  ttt cat cag      3069
caa gtt  tgg aag aac ccc aca  ttt ttc ctg cgc gtc  atc tct gac      3114
acg gcc  tcc ctc tgc tac tcc  atc ctg aaa gcc aag  aac gca ggg      3159
atg tcg  ctg ggg gcc aag ggc  gcc gcc ggc cct ctg  ccc tcc gag      3204
gcc gtg  cag tgg ctg tgc cac  caa gca ttc ctg ctc  aag ctg act      3249
cga cac  cgt gtc acc tac gtg  cca ctc ctg ggg tca  ctc agg aca      3294
gcc cag  acg cag ctg agt cgg  aag ctc ccg ggg acg  acg ctg act      3339
gcc ctg gag gcc gca gcc aac ccg gca ctg ccc tca gac ttc aag 3384
acc atc ctg gac tga 3399

<210> 2
<211> 1132
<212> PRT
<213> Homo sapiens

<400> 2

<210>  3
<211>  1131
<212>  DNA
<213>  herpes simplex virus 1

<220>
<221>  CDS
<222>  (1)..(1128)
<223>

<400>  3
atg gct tcg tac ccc tgc cat caa cac gcg tct gcg ttc gac cag gct        48
Met Ala Ser Tyr Pro Cys His Gln His Ala Ser Ala Phe Asp Gln Ala
1               5                   10                  15

gcg cgt tct cgc ggc cat agc aac cga cgt acg gcg ttg cgc cct cgc        96
Ala Arg Ser Arg Gly His Ser Asn Arg Arg Thr Ala Leu Arg Pro Arg
            20                  25                  30

cgg cag caa gaa gcc acg gaa gtc cgc ctg gag cag aaa atg ccc acg       144
Arg Gln Gln Glu Ala Thr Glu Val Arg Leu Glu Gln Lys Met Pro Thr
        35                  40                  45

cta ctg cgg gtt tat ata gac ggt cct cac ggg atg ggg aaa acc acc       192
Leu Leu Arg Val Tyr Ile Asp Gly Pro His Gly Met Gly Lys Thr Thr
    50                  55                  60

acc acg caa ctg ctg gtg gcc ctg ggt tcg cgc gac gat atc gtc tac       240
Thr Thr Gln Leu Leu Val Ala Leu Gly Ser Arg Asp Asp Ile Val Tyr
65                  70                  75                  80

gta ccc gag ccg atg act tac tgg cag gtg ctg ggg gct tcc gag aca       288
Val Pro Glu Pro Met Thr Tyr Trp Gln Val Leu Gly Ala Ser Glu Thr
                85                  90                  95

atc gcg aac atc tac acc aca caa cac cgc ctc gac cag ggt gag ata       336
Ile Ala Asn Ile Tyr Thr Thr Gln His Arg Leu Asp Gln Gly Glu Ile
            100                 105                 110

tcg gcc ggg gac gcg gcg gtg gta atg aca agc gcc cag ata aca atg       384
Ser Ala Gly Asp Ala Ala Val Val Met Thr Ser Ala Gln Ile Thr Met
        115                 120                 125

ggc atg cct tat gcc gtg acc gac gcc gtt ctg gct cct cat atc ggg       432
Gly Met Pro Tyr Ala Val Thr Asp Ala Val Leu Ala Pro His Ile Gly
    130                 135                 140

ggg gag gct ggg agc tca cat gcc ccg ccc ccg gcc ctc acc ctc atc       480
Gly Glu Ala Gly Ser Ser His Ala Pro Pro Pro Ala Leu Thr Leu Ile
145                 150                 155                 160

ttc gac cgc cat ccc atc gcc gcc ctc ctg tgc tac ccg gcc gcg cga       528
Phe Asp Arg His Pro Ile Ala Ala Leu Leu Cys Tyr Pro Ala Ala Arg
                165                 170                 175

tac ctt atg ggc agc atg acc ccc cag gcc gtg ctg gcg ttc gtg gcc       576
Tyr Leu Met Gly Ser Met Thr Pro Gln Ala Val Leu Ala Phe Val Ala
            180                 185                 190

ctc atc ccg ccg acc ttg ccc ggc aca aac atc gtg ttg ggg gcc ctt       624
Leu Ile Pro Pro Thr Leu Pro Gly Thr Asn Ile Val Leu Gly Ala Leu

195                 200                 205

ccg gag gac aga cac atc gac cgc ctg gcc aaa cgc cag cgc ccc ggc       672
Pro Glu Asp Arg His Ile Asp Arg Leu Ala Lys Arg Gln Arg Pro Gly
    210                 215                 220

gag cgg ctt gac ctg gct atg ctg gcc gcg att cgc cgc gtt tac ggg       720
Glu Arg Leu Asp Leu Ala Met Leu Ala Ala Ile Arg Arg Val Tyr Gly
225                 230                 235                 240

ctg ctt gcc aat acg gtg cgg tat ctg cag ggc ggc ggg tcg tgg cgg       768
Leu Leu Ala Asn Thr Val Arg Tyr Leu Gln Gly Gly Gly Ser Trp Arg
                245                 250                 255

gag gat tgg gga cag ctt tcg ggg acg gcc gtg ccg ccc cag ggt gcc       816
Glu Asp Trp Gly Gln Leu Ser Gly Thr Ala Val Pro Pro Gln Gly Ala
            260                 265                 270

gag ccc cag agc aac gcg ggc cca cga ccc cat atc ggg gac acg tta       864
Glu Pro Gln Ser Asn Ala Gly Pro Arg Pro His Ile Gly Asp Thr Leu
        275                 280                 285

ttt acc ctg ttt cgg gcc ccc gag ttg ctg gcc ccc aac ggc gac ctg       912
Phe Thr Leu Phe Arg Ala Pro Glu Leu Leu Ala Pro Asn Gly Asp Leu
    290                 295                 300

tac aac gtg ttt gcc tgg gcc ttg gac gtc ttg gcc aaa cgc ctc cgt       960
Tyr Asn Val Phe Ala Trp Ala Leu Asp Val Leu Ala Lys Arg Leu Arg
305                 310                 315                 320

ccc atg cac gtc ttt atc ctg gat tac gac caa tcg ccc gcc ggc tgc      1008
Pro Met His Val Phe Ile Leu Asp Tyr Asp Gln Ser Pro Ala Gly Cys
                325                 330                 335

cgg gac gcc ctg ctg caa ctt acc tcc ggg atg gtc cag acc cac gtc      1056
Arg Asp Ala Leu Leu Gln Leu Thr Ser Gly Met Val Gln Thr His Val
            340                 345                 350

acc acc ccc ggc tcc ata ccg acg atc tgc gac ctg gcg cgc acg ttt      1104
Thr Thr Pro Gly Ser Ile Pro Thr Ile Cys Asp Leu Ala Arg Thr Phe
        355                 360                 365

gcc cgg gag atg ggg gag gct aac tga                                  1131
Ala Arg Glu Met Gly Glu Ala Asn
    370                 375


<210>  4
<211>  376
<212>  PRT
<213>  herpes simplex virus 1

<400>  4

Met Ala Ser Tyr Pro Cys His Gln His Ala Ser Ala Phe Asp Gln Ala
1               5                   10                  15


Ala Arg Ser Arg Gly His Ser Asn Arg Arg Thr Ala Leu Arg Pro Arg
            20                  25                  30

Arg Gln Gln Glu Ala Thr Glu Val Arg Leu Glu Gln Lys Met Pro Thr
        35                  40                  45

Leu Leu Arg Val Tyr Ile Asp Gly Pro His Gly Met Gly Lys Thr Thr
    50                  55                  60

Thr Thr Gln Leu Leu Val Ala Leu Gly Ser Arg Asp Asp Ile Val Tyr
65                  70                  75                  80

Val Pro Glu Pro Met Thr Tyr Trp Gln Val Leu Gly Ala Ser Glu Thr
                85                  90                  95

Ile Ala Asn Ile Tyr Thr Thr Gln His Arg Leu Asp Gln Gly Glu Ile
            100                 105                 110

Ser Ala Gly Asp Ala Ala Val Val Met Thr Ser Ala Gln Ile Thr Met
        115                 120                 125

Gly Met Pro Tyr Ala Val Thr Asp Ala Val Leu Ala Pro His Ile Gly
    130                 135                 140

Gly Glu Ala Gly Ser Ser His Ala Pro Pro Pro Ala Leu Thr Leu Ile
145                 150                 155                 160

Phe Asp Arg His Pro Ile Ala Ala Leu Leu Cys Tyr Pro Ala Ala Arg
                165                 170                 175

Tyr Leu Met Gly Ser Met Thr Pro Gln Ala Val Leu Ala Phe Val Ala
            180                 185                 190

Leu Ile Pro Pro Thr Leu Pro Gly Thr Asn Ile Val Leu Gly Ala Leu
        195                 200                 205

Pro Glu Asp Arg His Ile Asp Arg Leu Ala Lys Arg Gln Arg Pro Gly
    210                 215                 220

Glu Arg Leu Asp Leu Ala Met Leu Ala Ala Ile Arg Arg Val Tyr Gly
225                 230                 235                 240

Leu Leu Ala Asn Thr Val Arg Tyr Leu Gln Gly Gly Gly Ser Trp Arg
                245                 250                 255

Glu Asp Trp Gly Gln Leu Ser Gly Thr Ala Val Pro Pro Gln Gly Ala
            260                 265                 270

Glu Pro Gln Ser Asn Ala Gly Pro Arg Pro His Ile Gly Asp Thr Leu
        275                 280                 285

Phe Thr Leu Phe Arg Ala Pro Glu Leu Leu Ala Pro Asn Gly Asp Leu
    290                 295                 300

Tyr Asn Val Phe Ala Trp Ala Leu Asp Val Leu Ala Lys Arg Leu Arg
305                 310                 315                 320

Pro Met His Val Phe Ile Leu Asp Tyr Asp Gln Ser Pro Ala Gly Cys
                325                 330                 335

Arg Asp Ala Leu Leu Gln Leu Thr Ser Gly Met Val Gln Thr His Val
            340                 345                 350

Thr Thr Pro Gly Ser Ile Pro Thr Ile Cys Asp Leu Ala Arg Thr Phe
        355                 360                 365

Ala Arg Glu Met Gly Glu Ala Asn
    370                 375

<210>  5
<211>  31
<212>  DNA
<213>  Artificial

<220>
<223>  TK Forward primer

<400>  5
ggagaattca tggcttcgta cccctgccat c                                    31

<210>  6
<211>  31
<212>  DNA
<213>  Artificial

<220>
<223>  TK Reverse primer

<400>  6
ggagcggccg ctcagttagc ctcccccatc t                                    31

<210>  7
<211>  20
<212>  DNA
<213>  Artificial

<220>
<223>  DLL1 Forward primer

<400>  7

tgcaggagtt cgtcaacaag                                                    20


<210>  8
<211>  20
<212>  DNA
<213>  Artificial

<220>
<223>  DLL1 Reverse primer

<400>  8
tccgtagtag tgttcgtcac                                                    20


<210>  9
<211>  24
<212>  DNA
<213>  Artificial

<220>
<223>  DLL4 Forward primer

<400>  9
cctgcattgt gaacacagca cctt                                               24


<210>  10
<211>  24
<212>  DNA
<213>  Artificial

<220>
<223>  DLL4 Reverse primer

<400>  10
agttcacagt aggtgcccgt gaat                                               24


<210>  11
<211>  20
<212>  DNA
<213>  Artificial

<220>
<223>  E-cadherin Forward primer

<400>  11
cgacccaacc caagaatcta                                                    20


<210>  12
<211>  20
<212>  DNA
<213>  Artificial

<220>
<223>  E-cadherin Reverse primer

<400>  12
aggctgtgcc ttcctacaga                                                    20

<210>  13
<211>  24
<212>  DNA
<213>  Artificial

<220>
<223>  GAPDH Forward primer

<400>  13
tccagaacat catccctgcc tcta                                              24


<210>  14
<211>  25
<212>  DNA
<213>  Artificial

<220>
<223>  GAPDH Reverse primer

<400>  14
tgttgaagtc agaggagacc acctg                                             25

## Description

### TECHNICAL FIELD

The present invention relates to a feeder cell for target cell induction and to a production process for a target cell sheet using the feeder cell for target cell induction.

### BACKGROUND ART

Corneal epithelial stem cells are considered to reside in the corneal limbus. When the stem cells in the corneal limbus are damaged by severe chemical injuries and burn injuries, or intractable diseases such as Stevens-Johnson syndrome and ocular pemphigoid, the surface of the corneal epithelium may be covered with conjunctival tissue, which may result in severe visual impairment. Therapeutic methods for such impairment include corneal limbal transplantation and the like, but these methods entail problems such as donor shortage and allograft rejection.

In recent years, therapy has been attempted by transplanting corneal epithelial cells cultured from autologous cells, i.e., by transplanting an epithelial cell sheet produced from autologous cells such as corneal epithelial cells and oral mucosal cells. This method has already been clinically applied as a method for overcoming the above problems, including donor shortage and rejection.

Known methods for producing such an epithelial cell sheet include a method containing co-culturing autologous cells such as corneal epithelial cells and oral mucosal cells with feeder cells, which are usually mouse-derived fibroblasts (3T3 cells) that have been inactivated with mitomycin C, on a substrate such as an amnion and collagen or on a temperature-responsive culture surface (for example, see WO 2004/078225, WO 2006/003818, and WO 2006/075602).

However, since mouse-derived cells are used for co-culturing in this method, there may be a risk of infection from and contamination with heterologous cells.

Known methods for solving the above problems such as contamination with heterologous cells include a method for producing a corneal epithelial cell sheet using human fibroblasts as feeder cells (see WO 2005/087285).

However, even when a corneal epithelial cell sheet is produced using human fibroblasts, there is difficulty in removing the feeder cells.

### SUMMARY OF INVENTION

### TECHNICAL PROBLEM

An object of the present invention is to provide a feeder cell for target cell induction, a production process for a target cell sheet using the feeder cell, a process for determining the function of a candidate inducing factor for a target cell, a process for culturing a target cell on a feeder cell transduced with the gene of a candidate inducing factor, and a process for transplanting the target cell.

### SOLUTION TO PROBLEM

The inventors have conducted extensive research to solve the above problems and, as a result, found out that introduction of an immortalizing gene and a suicide gene into a cell, preferably a cell derived from the same species as that of a target cell from which a sheet is to be made and also anatomically contiguous to human corneal epithelial cells, will give a feeder cell having the following excellent advantages: the feeder cell can be maintained through long-term passage; the feeder cell can be killed with a selective drug when it becomes unnecessary; and since the feeder cell can be killed with a selective drug, adherent culture of the feeder cell with a target cell can be performed. Furthermore, the inventors have also found out that by introducing a marker gene into the feeder cell, the feeder cell can be visually observed when a target cell is contaminated with the feeder cell. Based on these findings, the inventors conducted further research and completed the present invention.

That is, the present invention relates to
(1) a feeder cell for target cell induction, wherein the feeder cell is transduced with an immortalizing gene and a suicide gene;
(2) the feeder cell for target cell induction according to the above (1), wherein the feeder cell is derived from a cell that is contiguous to the target cell;
(3) the feeder cell for target cell induction according to the above (1), wherein the feeder cell is derived from a human corneal stromal cell;
(4) the feeder cell for target cell induction according to the above (1), wherein the feeder cell is derived from a human dermal fibroblast;
(5) the feeder cell for target cell induction according to any of the above (1) to (4), wherein the immortalizing gene is an hTERT gene;
(6) the feeder cell for target cell induction according to any of the above (1) to (5), wherein the suicide gene is herpes simplex virus type 1 thymidine kinase gene;
(7) the feeder cell for target cell induction according to any of the above (1) to (6), wherein the feeder cell is further transduced with a marker gene;
(8) the feeder cell for target cell induction according to the above (7), wherein the marker gene is an EGFP gene;
(9) the feeder cell for target cell induction according to any of the above (1) to (8), wherein the target cell is a human-derived cell that requires a feeder cell for cell culture;
(10) the feeder cell for target cell induction according to any of the above (1) to (9), wherein the target cell is a human corneal epithelial cell, a human corneal endothelial cell, a human conjunctival epithelial cell, or a human kidney-derived cell;
(11) the feeder cell for target cell induction according to any of the above (1) to (9), wherein the feeder cell is further transduced with the gene of a stimulating factor for the target cell;
(12) the feeder cell for target cell induction according to the above (11), wherein the stimulating factor for the target cell is one or more human cell adhesion molecules selected from a cadherin superfamily, an integrin family, an immunoglobulin superfamily, a secretin family, a neuroligin, a neurexin, and a cell surface proteoglycan; one or more growth factors selected from an epidermal growth factor (EGF) family, a vascular endothelial cell growth factor (VEGF) family, an insulin-like growth factor (IGF) family, and a fibroblast growth factor (FGF) family; or one or more Notch ligands selected from a Jagged family and a Delta-like member;
(13) a production process for a target cell sheet, comprising the steps of
   (a) culturing a feeder cell for target cell induction according to any of the above (1) to (12) on a cell culture carrier, and
   (b) culturing and growing the target cell by seeding the target cell on the cell culture carrier of the step (a) and then by allowing the target cell to stand;
(14) the production process according to the above (13), wherein the process further comprises the step of (c) separating the feeder cell for target cell induction from the target cell sheet;
(15) the production process according to the above (14), wherein the separation in the step (c) is carried out by treating the target cell sheet with a precursor of a suicide gene-inducible toxic substance;
(16) the production process according to the above (15), wherein the suicide gene is herpes simplex virus type 1 thymidine kinase gene, and the precursor of a suicide gene-inducible toxic substance is ganciclovir;
(17) the production process according to any of the above (13) to (16), wherein the target cell is a human corneal epithelial cell, a human corneal endothelial cell, a human conjunctival epithelial cell, or a human kidney-derived cell;
(18) the production process according to any of the above (13) to (17), wherein human serum is used in the step (a) or (b);
(19) a process for determining the function of a candidate inducing factor for a target cell, comprising the steps of
   (a') culturing a feeder cell according to any of the above (1) to (10) on a cell culture carrier,
   (b') transducing a feeder cell according to any of the above (1) to (10) with the gene of the candidate inducing factor for the target cell, and culturing the obtained candidate inducing factor-transduced feeder cell on a cell culture carrier,
   (c') culturing and growing the target cell by seeding the target cell on each of the cell culture carriers of the above (a') and (b') and then by allowing the target cell to stand, and
   (d') evaluating the cells cultured on each of the cell culture carriers;
(20) a process for culturing a target cell, comprising the steps of
   (i) transducing a feeder cell of the present invention with the gene of a candidate inducing factor for the target cell,
   (ii) culturing the candidate inducing factor-transduced feeder cell obtained in the step (i) on a cell culture carrier, and
   (iii) culturing and growing the target cell by seeding the target cell on the cell culture carrier of the step (ii) and then by allowing the target cell to stand; and (21) a process for transplanting a target cell, comprising the step of transplanting a target cell sheet obtained by the production process for a target cell sheet according to any of the above (13) to (18) into an intended site.

### ADVANTAGEOUS EFFECTS OF INVENTION

The use of the feeder cell of the present invention enables culture of a target cell and production of a cell sheet of a target cell. The use of the feeder cell of the present invention also enables simple and easy culture of human corneal endothelial cells, human conjunctival epithelial cells, human kidney-derived cells, human iPS cells, human ES cells, or the like.

### BRIEF DESCRIPTION OF DRAWINGS

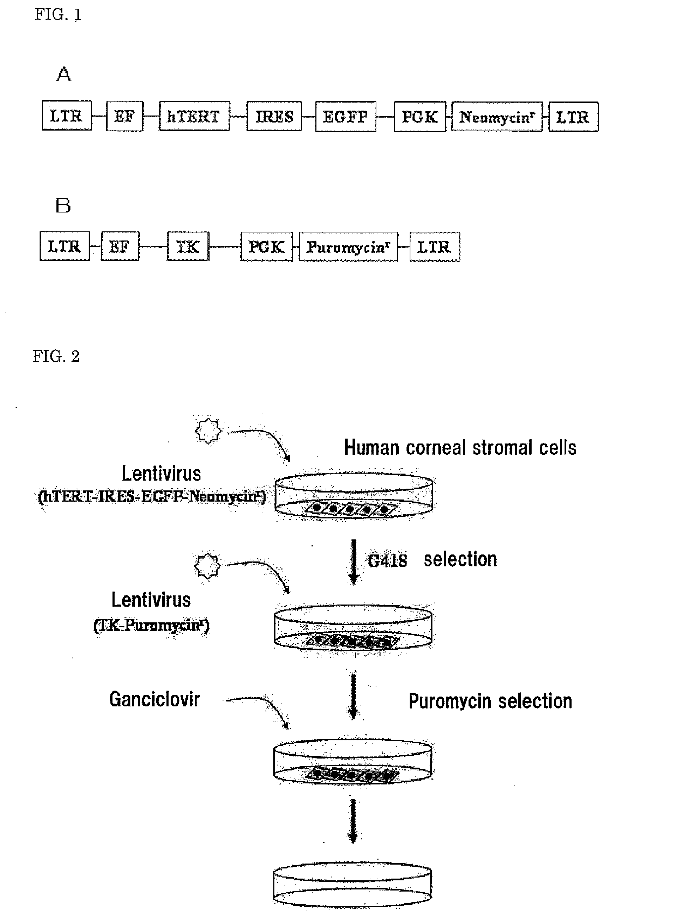

Fig. 1 shows the construct of the recombinant lentiviral vector that was used to transduce human corneal stromal cells in Example 1. Fig. 1A shows the lentiviral vector in Example 1, into which the hTERT gene, the IRES-EGFP gene, and the neomycin-resistance gene were introduced. Fig. 1B shows the lentiviral vector in Example 1, into which the HSV-TK gene shown in SEQ ID: NO. 3 and the puromycin-resistance gene were introduced. In the Figure, LTR represents a long terminal repeat, EF represents a human elongation factor (1α subunit) gene promoter, hTERT represents a human telomerase reverse transcriptase gene, IRES represents an internal ribosome entry site, EGFP represents an enhanced green fluorescent protein gene, PGK represents a phosphoglycerate kinase promoter, Neamycin^{r} represents a neomycin-resistance gene, TK represents HSV thymidine kinase, and Puromycin^{r} represents a puromycin-resistance gene.
Fig. 2 shows the schematic view of transfection of human corneal stromal cells.
Fig. 3 shows the susceptibilities of human corneal stromal cells to ganciclovir treatment. The left figure shows changes in cell number (%) of the hTERT-transduced human corneal stromal cells, which were not transduced with the HSV-TK gene, after ganciclovir treatment. The right figure shows changes in cell number (%) of the hTERT- and HSV-TK-transduced human corneal stromal cells after ganciclovir treatment. The values in the graphs represent average ± standard deviation (n = 3).
Fig. 4A shows the schematic view of the production process for a human corneal epithelial cell sheet. Fig. 4B shows, from the top, the feeder cells, the human corneal epithelial cells, and the human corneal epithelial cell sheet, all of which were obtained in Example 2.
Fig. 5 shows the photographs of the cells cultured on the transduced feeder cells of the present invention. The target cell in each photograph was human conjunctival epithelial cells in A, human corneal endothelial cells in B, and human kidney-derived cells (293T cells) in C.
Fig. 6 shows the human corneal epithelial cells obtained by culture using human serum.

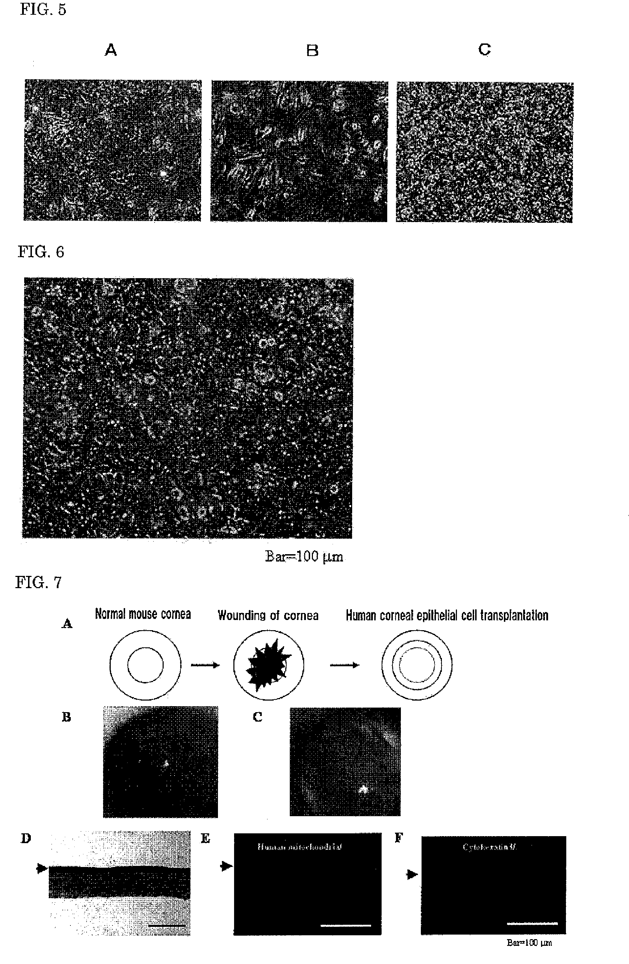

Fig. 7 shows transplantation of a human corneal epithelial cell sheet in a mouse model of corneal damage. Fig. 7A shows a transplantation procedure of a cultured corneal epithelial cell sheet. Fig. 7B shows a mouse cornea on the 15th day after the corneal epithelial cell layer was completely removed by exposing the cornea surface including the limbus to 50% ethanol. Fig. 7C shows a mouse cornea on the 7th day after transplantation of the corneal epithelial cell sheet obtained in Example 8. The transplantation was carried out immediately after the corneal epithelial cell layer was completely removed by exposing the cornea surface including the limbus to 50% ethanol. D shows the result of microscopic observation of HE staining of the corneal epithelial cells that formed 3 to 5 layers after the above transplantation. The arrow indicates the part in which the corneal epithelial cells formed layers. E shows the result of human mitochondria (green) and DAPI (blue) staining of the corneal epithelial cells after the transplantation. The arrow indicates the human corneal epithelial cells. F shows the result of cytokeratin 3 (green) and DAPI (blue) staining of the corneal epithelial cells after the transplantation. The arrow indicates the human corneal epithelial cells.

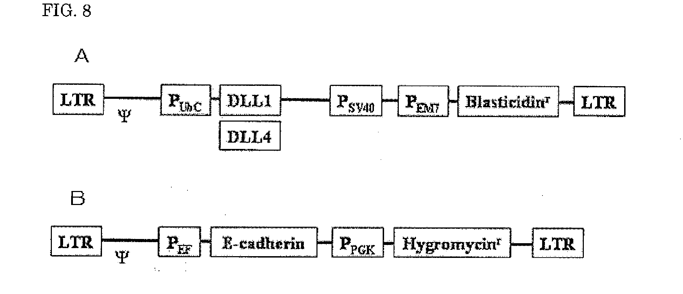

Fig. 8 shows the construct of the recombinant lentiviral vector that is used to transduce human corneal stromal cells. Fig. 8A shows the DLL1- and blasticidin-resistance gene expression lentiviral vector, or the DLL4- and blasticidin-resistance gene expression lentiviral vector in Example 9. Fig. 8B shows the E-cadherin- and hygromycin-resistance gene expression lentiviral vector in Example 9. In the Figure, LTR represents a long terminal repeat, Ψ represents a packaging signal, P_{UbC} represents a human ubiquitin C gene promoter, DLL1 represents the Delta-like 1 gene, DLL4 represents the Delta-like 4 gene, P_{SV40} represents an SV40 gene promoter, EM7 represents an EM7 gene promoter for expression in *Escherichia coli*, Blasticidin^{r} represents a blasticidin-resistance gene, P_{EF} represents a human elongation factor (1α subunit) gene promoter, E-cadherin represents the E-cadherin gene, P_{PGK} represents a phosphoglycerate kinase gene promoter, and Hygromycin^{r} represents a hygromycin-resistance gene.
Fig. 9 shows the results of RT-PCT. Fig. 9A shows the result of RT-PCT of the hTERT+TK-transduced human corneal stromal cells, and Fig. 9B shows the result of RT-PCT of the DLL1+, DLL4+, or E-cadherin+, hTERT+TK-transduced human corneal stromal cells.
Fig. 10 shows the colony forming rate of the human corneal epithelial cells in Example 10. The values (%) of the graph represent average ± standard deviation (n = 3).
Fig. 11 shows the photographs of colony formation of human corneal epithelial cells cultured on transduced human corneal stromal cells and on transduced human dermal fibroblasts. Fig. 11A shows the result of colony formation of the human corneal epithelial cells cultured on the hTERT+TK-transduced human corneal stromal cells, and Fig. 11B shows the result of colony formation of the human corneal epithelial cells cultured on the hTERT+TK-transduced human dermal fibroblasts.
Fig. 12 shows the photographs of the culture result of corneal endothelial cells cultured on transduced human corneal stromal feeder cells.

### DESCRIPTION OF EMBODIMENTS

A first embodiment of the present invention is a feeder cell line for target cell induction, wherein the feeder cell line is transduced with an immortalizing gene and a suicide gene. The first embodiment will be explained below.

The feeder cell line for target cell induction of the present invention can be produced by introducing DNA sequences encoding an immortalizing gene and a suicide gene into a host cell with the use of an expression vector.

The "immortalizing gene" in the present invention means a gene that gives cells the ability to grow and live indefinitely. The immortalizing gene is not specifically limited, and examples thereof include oncogenes such as c-myc and ras; adenovirus E1A; the large T antigen gene derived from SV40 (the SV40 large T antigen gene); the large T antigen gene derived from polyomavirus; the E6 gene and the E7 gene of papillomavirus type 16 (HPV16); and the human telomerase reverse transcriptase (hTERT) gene. Examples of the immortalizing gene also include a gene derived from telomerase, and a gene regulating the expression or activity of telomerase (for example, the myc gene, which is said to increase the activity of telomerase). Among these, the SV40 large T antigen gene or the human telomerase reverse transcriptase gene is preferable. The SV40 large T antigen gene is a known tumor antigen (T antigen) gene of a DNA tumor virus. The hTERT gene is derived from the TERT gene of a normal cell. In the present invention, the human telomerase (hTERT) gene is especially preferable. The SV40 large T antigen gene is not specifically limited, but a wild-type SV40 T antigen gene (SV40 TAg; Genbank NC_001669) may be used and a temperature-sensitive mutant SV40 tsT antigen gene may also be used. The human telomerase reverse transcriptase (hTERT) is not specifically limited, but full-length human telomerase reverse transcriptase isoform 1 (hTERT; Genbank NM_003219; SEQ ID: NO. 1; the amino acid sequence is shown in SEQ ID: NO. 2) is preferable. In mammalian somatic cells, a telomere, which resides at the end of the chromosome, is shortened at every cell division and, after a fixed number of divisions, cell growth will stop. The telomerase reverse transcriptase is an enzyme that is responsible for maintaining the length of a telomere, and it is known that by introducing the telomerase reverse transcriptase gene into a cell, the number of cell division can be extended and the cell would become immortal (Counter et al., Proc Natl Acad Sci USA. 95, 14723-14728: 1998). The above immortalizing gene may be used alone or in a combination of two or more.

The "suicide gene" in the present invention means a gene encoding a bacteria- or virus-derived enzyme that has the function of metabolizing a low-activity antiviral agent and converting it into a substance with a strong cytotoxic activity. When such a suicide gene is introduced into a cell, the cell will be selectively killed by administration of an antiviral agent (hereinafter sometimes referred to as "a precursor of a suicide gene-inducible toxic substance"). Therefore, when the suicide gene is introduced into the feeder cell, the feeder cell can be selectively removed by administration of an antiviral agent according to the purpose. A combination of the suicide gene and the antiviral agent is not specifically limited, and examples of the combination include a combination of herpes simplex virus type 1 thymidine kinase (HSV-TK, hereinafter also abbreviated to "TK") gene and ganciclovir (GCV) or acyclovir (ACV), which is clinically used as an antiviral agent; and a combination of the cytosine deaminase gene of *Escherichia coli* and an antimicrobial agent 5-fluorocytosine (Elion GB. Am. J. Med. 73, 7, 1982; Craig AM. Proc. Acad. Sci. USA 89, 33, 1992). The combination of the HSV-TK gene (SEQ ID: NO. 3) and ganciclovir or acyclovir is preferable in that the efficacy of the combination has already proved in an animal experiment (Moolten FL, Wells JM: J Natl Cancer Inst. 82: 297-300, 1990; Culver KW, Ram Z, Wallbridge S, Ishii H, Oldfield EH. Science 1992; 256: 1550) and that the efficacy has also been confirmed in clinical application to gene therapy for a human prostate cancer. The HSV-TK gene encodes HSV-TK, which is a thymidine kinase unique to a virus and consists of 376 amino acids (SEQ ID: NO. 4). The substrate specificity of HSV-TK is different from that of a thymidine kinase in human cells and HSV-TK has the activity of phosphorylating an analogue of guanosine, acyclovir (ACV) or ganciclovir (GCV) (Moolten FW and Wells JM. Tumor chemosensitivity conferred by inserted herpes thymidine kinase genes: paradigm for a prospective cancer control strategy. Cancer Res 46: 5276-5281, 1986.; Reardon JE. Herpes simplex virus type 1 and human DNA polymerase interactions with 2'-deoxyguanosine 5'-triphosphate analogues: kinetics of in corporation into DNA and induction of inhibition. J Biol Chem 264: 19039-19044, 1989.). Acyclovir or ganciclovir exhibits no toxicity to a cell that does not express any kinase for which acyclovir or ganciclovir is a substrate. In a cell that expresses HSV-TK as a result of virus infection, transduction with the HSV-TK gene, or the like, acyclovir or ganciclovir is phosphorylated to a monophosphate and the monophosphate is further converted to a di- or tri-phosphate by endogenous guanylate kinase and thymidine kinase. The end product, the triphosphate, inhibits DNA polymerase and terminates DNA elongation, thereby strongly damaging and eventually killing the cell. In this way, HSV-TK in combination with acyclovir or ganciclovir exhibits the biological activity.

The feeder cell line for target cell induction of the present invention is not specifically limited, but the feeder cell line more preferably has the ability of expressing a marker protein, which enables visual distinction between cells. Examples of the marker gene contained in the feeder cell line for target cell induction of the present invention include the EYFP (enhanced yellow fluorescent protein) gene, the ECFP (enhanced cyan fluorescent protein) gene, the DsRed1 gene, and the EGFP (enhanced green fluorescent protein) gene. Among these, the EGFP gene is preferable. The existence of the feeder cell for target cell induction used in the present invention can be visually confirmed by expression of the marker protein, which reduces the risk of contamination with the feeder cell at the time of transplantation.

The expression vector used in the present invention is not specifically limited, and examples thereof include a plasmid vector and a viral vector. A viral vector is preferable in that an intended cell can be efficiently transduced just through the addition of the filtrate of the culture supernatant of a cell producing a recombinant viral vector to the intended cell. Examples of the plasmid vector include pBR322 and pGEM. Examples of the viral vector include a retrovirus such as Moloney murine leukemia virus and lentivirus, adenovirus, herpesvirus, adeno-associated virus, parvovirus, Semliki Forest virus, vaccinia virus, and Sendai virus. Preferred is lentivirus, adenovirus, or herpesvirus. The construct of the above expression vector, including the kind of a promoter, is not limited as long as the construct does not prevent the effects of the present invention. Examples of a promoter contained in the above expression vector include an SV40 early promoter, a Rous sarcoma virus (RSV) promoter, MuMLV LTR, human CMV, human EF1α, human UbC, and PGK.

The method for introducing the above genes is not specifically limited, and examples of the method include a virus system with the use of lentivirus (ViraPower Lentiviral Expression System (Invitrogen)), a retrovirus (RetroMax Retroviral Expression System (IMGENEX), Retrovirus Constructive System (Takara Bio, Inc.), or pSilencer Retro System (Ambiom)), or adenovirus (AdMax system (Microbix), or ViraPower Adenoviral Expression System (Invitrogen)); and the HVJ liposome method.

The order of introduction of the above immortalizing gene and suicide gene, and as needed the marker gene, is not specifically limited and the introduction can be started with any of these genes. However, the immortalizing gene is preferably introduced first due to ease of culture.

The host cell into which the above genes are introduced with the use of an expression vector is usually a cell derived from the same species as that of a target cell. The host cell is preferably a cell that is anatomically contiguous to a target cell, but is not specifically limited thereto. For example, when the target cell is a human corneal epithelial cell or a human corneal endothelial cell, a suitable host cell may be exemplified by a human corneal Stromal cell. A human corneal stromal cell can be obtained by, for example, isolating corneal tissue from a harvested ocular tissue, cutting the corneal tissue into pieces (about 1 mm square) with scissors or the like, and digesting the obtained corneal fragments with collagenase type IV. The corneal stromal cells thus obtained are suspended in a suitable liquid medium containing 10 to 20% fetal calf serum or calf serum. Examples of the liquid medium include Eagle's MEM medium, Dulbecco's Modified Eagle's MEM medium, Ham's F12 medium, and Katsuta medium DM-160. The cells are then cultured in a CO₂ incubator and subjected to transfection. The host cell, as mentioned above, is not specifically limited as long as the host cell is derived from the same species as that of a target cell. For example, as the host cell, a skin cell can be used, and preferably a dermal fibroblast can be used. For example, a transduced feeder cell derived from a human skin cell can be used to produce a human corneal epithelial cell sheet or the like.

The method for establishing and maintaining a cell line transduced with the above genes is not specifically limited as long as the method is a common animal cell culture technology. The cell line may be cultured in a liquid medium such as Eagle' s MEM medium, Dulbecco's Modified Eagle's MEM medium (DMEM), Ham' s F12 medium, and Katsuta medium Dam-160. As the conditions such as culture temperature, medium pH and CO₂ concentration, culture conditions commonly used for animal cell culture are employed as appropriate. The medium is replaced with a fresh medium once every 2 to 3 days, and when cell growth reaches saturation, the cells are subcultured. The cells may be a heterogeneous cell population, but preferably the cells is a monoclonal cell population, which can be obtained by continuing to culture the cells even after cell growth once stops, and separating a cell population that starts to grow again. The separation method for the monoclonal cell population is not specifically limited, but, for example, the separation can be carried out as follows. A small-diameter filter paper in which a trypsin and EDTA solution is absorbed is placed on an intended clonal cell population and left to stand. The filter paper along with the clonal cell population is then removed from a culture vessel. The filter paper to which the clonal cell population adheres is transferred to another culture vessel. Alternatively, a clonal cell population can be separated by colony pick up, a limiting dilution method, a method using a cell sorter, or the like.

A target cell that the feeder cell of the present invention is intended to induce is not specifically limited as long as the target cell is a cell that requires a feeder cell for cell culture, but preferably the target cell is a human-derived cell that requires a feeder cell for cell culture. In particular, suitable examples of the target cell include ocular epithelial cells such as a human corneal epithelial cell and a human conjunctival epithelial cell; a human corneal endothelial cell; and stem cells such as a human iPS cell (induced pluripotent stem cell) and a human ES cell (embryonic stem cell). Since an established cell line can be easily grown, an established cell line derived from human kidney or the like is also suitable as the target cell.

The feeder cell of the present invention can also be forced to express a stimulating factor for a target cell. The stimulating factor for a target cell is not specifically limited as long as it is specific to the kind of a target cell. Examples of such a stimulating factor for a target cell include a human cell adhesion molecule such as the cadherin superfamily, the integrin family, the immunoglobulin superfamily, the secretin family, a neuroligin, a neurexin, and a cell surface proteoglycan; a growth factor such as the epidermal growth factor (EGF) family, the vascular endothelial cell growth factor (VEGF) family, the insulin-like growth factor (IGF) family, and the fibroblast growth factor (FGF) family; and a Notch ligand such as the Jagged family and a Delta-like member. By introducing the gene of the above stimulating factor into the feeder cell of the present invention, the feeder cell can be forced to express the stimulating factor on the cell surface. The feeder cell can also be forced to express a variety of factors that can affect cell-cell interactions, including the above stimulating factor, on the cell surface. The expression of such a factor enables the feeder cell to affect growth and differentiation of a target cell such as a corneal cell or an iPS cell and to control a target cell that has been difficult to control. For example, a cytostatic activity can be controlled using a feeder cell that is forced to express DLL protein. The expression of the above growth factor can improve growth-maintaining culturing effects including a cell growth-promoting effect and a normal cell form-maintaining effect.

The feeder cell of the present invention, as described above, can be a powerful tool in regenerative medicine, which is intended to appropriately control a cell that has been difficult to control until now. The gene of the above stimulating factor for a target cell is not specifically limited, and one or more of the genes can be introduced into the feeder cell of the present invention.

The cadherin superfamily includes classic cadherins and non-classic cadherins. Examples of the classic cadherins include E-cadherin, N-cadherin, P-cadherin, R-cadherin, M-cadherin, and VE-cadherin. Examples of the non-classic cadherins include desmocollin, desmoglein, T-cadherin, protocadherin, and Fat. The integrin family is a heterodimer molecule containing two distinct single-pass transmembrane proteins (α chain and β chain). There are 19 kinds of α chains and 8 kinds of β chains. There exist at least 25 kinds of αβ heterodimers. Most part of the molecule protrudes out of a cell and, to the intracellular domain, an actin filament binds via talin, vinculin, tensin, and α-actinin. Examples of a member of the integrin family include LFA-1, VLA1-5, and VLA-4. The immunoglobulin superfamily includes the immune system family and the nervous system family. Examples of a member of the immune system family include Ig, TCR, CD2, CD4, CD8, CD16 (FcγRIII), CD56 (NCAM-1), CD57, andMHC (class I and class II). Examples of a member of the nervous system family include ICAM-1. Examples of a member of the secretin family include E-secretin, P-secretin, and L-secretin. Examples of the neuroligin include neuroligin 1, neuroligin 2, and SynCAM1. Examples of the Delta-like member include DLL 1 (Delta-like 1), DLL 3

(Delta-like 3), and DLL 4 (Delta-like 4). The EGF family is not specifically limited, and examples of a member of the EGF family include EGF, TGF-α, amphiregulin (AR), HB-EGF, epiregulin (EPR), betacellulin (BTC), neuregulins 1-4 (NRG 1-4), and teratocarcinoma-derived growth factor (Cripto 1). The VEGF family is not specifically limited, and examples of a member of the VEGF family include VEGF-A, VEGF-B, VEGF-C, VEGF-D, VEGF-D, VEGF-E, PGF (placental growth factor)-1, and PIGF-2. The IGF family is not specifically limited, and examples of a member of the IGF family include EGF-1 and IGF-2. The FGF family is not specifically limited, and examples of a member of the FGF family include a basic fibroblast growth factor (bFGF) and an acidic fibroblast growth factor (aFGF). Examples of a member of the Jagged family include JAG1 and JAG2.

A second embodiment of the present invention is a production process for a target cell sheet, comprising the steps of
(a) culturing a feeder cell for target cell induction, prepared as described above, on a cell culture carrier, and
(b) culturing and growing the target cell by seeding the target cell on the cell culture carrier of the step (a) and then by allowing the target cell to stand.

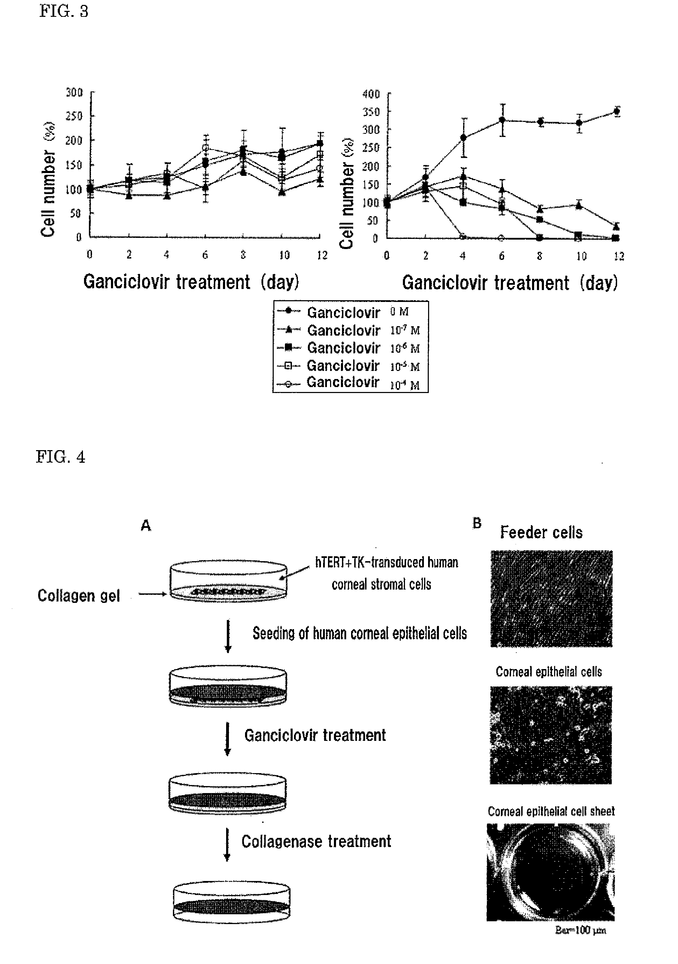

The second embodiment will be explained below. The production process is exemplified by cases where the target cell is a human corneal epithelial cell in Fig. 4A, which shows the schematic view of the production process of a human corneal epithelial cell sheet.

The cell culture carrier for the feeder cell in the step (a) is not specifically limited, but suitable examples thereof include a temperature-responsive cell culture surface, an amnion, and a collagen gel. The culture method for the feeder cell on the culture carrier for the feeder cell in the step (a) is not specifically limited, and examples of the method include a method containing seeding the feeder cell for target cell induction on the surface of a temperature-responsive cell culture surface, an amnion, or a collagen gel; and a method containing embedding the feeder cell for target cell induction in a collagen gel, and culturing the feeder cell. The temperature-responsive cell culture surface is not specifically limited, but suitable examples thereof include the temperature-responsive cell culture surface described in JP-2006-320304-A or JP-2008-271912-A. Examples of a marketed product of the temperature-responsive cell culture surface include UpCell (trade name)(CellSeed Inc.). The amnion or collagen gel functions as a culture substrate for a target cell. An "amnion" is a membrane that covers the innermost surface of a placenta in a womb of a mammal, and is composed of epithelial layer and a basement membrane that are formed on parenchymal tissue containing a large proportion of collagen. A preferred amnion is a human amnion. A human amnion can be harvested from, for example, a human fetal membrane, a placenta, or the like, which is obtained as the afterbirth after delivery. In particular, a human amnion can be prepared by treating and purifying an integrated unit which contains a human fetal membrane, a placenta, and an umbilical cord, and which is obtained immediately after delivery. As the treatment method and the purifying method, a known method such as a method described in JP-5-5698-A can be employed. That is, a human amnion can be prepared by peeling an amnion off a fetal membrane obtained after delivery, removing remained tissue from the amnion by physical treatment such as ultrasonic cleaning, enzyme treatment, and the like, and washing the amnion as needed.

A human amnion thus prepared can be cryopreserved until used. A human amnion can be frozen, for example, at -80°C in a mixed solution of DMEM and glycerol at an equivalent volume ratio. The cryopreservation not only improves handling but also is expected to reduce antigenicity.

An obtained amnion can be used as it is, but preferably the epithelium is removed from the amnion with curettage or the like. Removal of the epithelium will reduce antigenicity. A human amnion free from epithelium can be prepared by, for example, thawing a human amnion that has been cryopreserved as described above, weakening adhesion between cells with treatment using EDTA or a protease, and curetting epithelium with a cell scraper or the like. Furthermore, the amnion is preferably subjected to drying beforehand. Examples of the drying method include freeze-drying, air-drying, vacuum drying, and reduced pressure drying. Among these, freeze-drying is preferably employed. This is because, in freeze-drying, the flexibility of the amnion will be hardly reduced.

The kind of a collagen used as a raw material for the collagen gel is not specifically limited, and type I collagen, type II collagen, type III collagen, type IV collagen, or the like can be used. Collagens that can be used also include a mixed collagen of different kinds. These collagens can be extracted and purified from connective tissue such as a skin and a cartilage of an animal including swine, cattle, sheep, or the like by an acid solubilization method, an alkali solubilization method, an enzyme solubilization method, or the like. For the purpose of reducing antigenicity, it is preferable to use a so-called atelocollagen, which is obtained by removing telopeptides with treatment using a degradative enzyme such as pepsin and trypsin. As a raw material for the collagen gel, a collagen derived from an amnion, in particular, a collagen derived from a human amnion may be used. The phrase "derived from an amnion" herein means "obtained from an amnion, in its broadest sense, as a starting material."

In cases where the above feeder cell (hereinafter sometimes referred to as the transduced feeder cell) is embedded in the collagen gel and then cultured, a collagen gel-embedded culture method can be used. The production process for the collagen gel in this case is exemplified as follows. For example, a neutralized collagen solution is prepared from type I collagen. This solution is poured into a culture vessel (for example, Culture Insert) and left to stand at room temperature for 10 minutes so that the solution is allowed to gel. Next, this gel is mixed with the above feeder cell in the logarithmic growth phase that has been cultured by the above process beforehand, and the mixture is allowed to gel again. Next, the cell is statically cultured. By this procedure, the collagen gel containing the feeder cell can be obtained.

In cases where the feeder cell is seeded on the surface of the collagen gel, an attached collagen gel culture method or a floating collagen gel culture method can be used. In the attached collagen gel culture method, a suspension of the feeder cell is seeded on a collagen gel that has been produced in a culture dish, and after the cell adheres to the gel, the cell is cultured in the same manner as in a monolayer culture method. In cases where serum or other physiologically active substances are added to the gel, these substances are mixed into a collagen mixed solution for gel preparation. However, addition of serum or the like to the collagen gel is not necessarily required because a medium rapidly permeates the gel. In the floating collagen gel culture method, the cell is cultured by the above attached collagen gel culture method, and after the cell adheres to the collagen gel and forms a monolayer, the gel is peeled off the culture dish and allowed to float in a medium while carrying the feeder cell. The gel can be peeled and allowed to float by running once the tip(s) of an injection needle or tweezers along the inner edge of a culture dish, and then pushing the tip(s) under the gel toward the center. The collagen gel can be produced using a marketed product, for example, Collagen Gel Culture Kit (Nitta Gelatin, Inc.).

In the step (a) , the conditions such as culture temperature, medium pH, and CO₂ concentration are not specifically limited, and culture conditions commonly used for animal cell culture are employed as appropriate. The feeder cell is usually cultured until it reaches confluency. For example, in cases where the feeder cell is a cell that forms multilayers as epithelial cells do, the feeder cell may be further cultured for about 1 to 2 weeks and allowed to form multilayers. In this way, the feeder cell having similar conditions to those in a living body can be obtained.

The above transduced feeder cell used in the step (a) is more preferably inactivated with mitomycin C or the like beforehand. This inactivation is intended to prevent inhibition of target cell growth by growth of the feeder cell in the following step (b) and then improve growth efficiency of the target cell. Such inactivation can also be carried out by radiation treatment or the like. After that, the feeder cell is seeded and cultured on an amnion or a collagen gel to form a layer on the surface of the amnion or the collagen gel.

In the step (b), the target cell can be cultured as needed by culture technology commonly used for animal cells, or alternatively the target cell can be obtained as a marketed product. For example, when the target cell is a corneal epithelial cell, the corneal epithelial cell can be obtained from corneal limbal tissue. That is, the corneal epithelial cell can be obtained by, for example, peeling and removing endothelial cells from corneal limbal tissue, excising a conjunctiva, preparing a single-cell suspension, storing the suspension in a nitrogen tank and then rapidly thawing the suspension at 37°C to give a corneal epithelial cell suspension, and subculturing the cells as needed. A medium that can be used for subculturing the cells include, for example, a serum free medium such as EpiLife™ (Cascade Biologics, Inc.) and MCDB153 medium (NISSUI PHARMACEUTICAL CO. , LTD.), and a medium produced by modifying the amino acid composition or the like of these media. For example, when the target cell is a corneal epithelial cell, the corneal epithelial cell is preferably prepared from a person (recipient) who will undergo transplantation of the corneal epithelium sheet. That is, the donor of the target cell is preferably identical with the recipient of the target cell sheet. Use of such an autologous cell can prevent immunological rejection.

In the step (b), the target cell is seeded (loaded) on the feeder cell cultured on the cell culture carrier in the step (a). For example, when the target cell is a human corneal epithelial cell, the corneal epithelial cell can be seeded at a cell density of, for example, preferably about 1 × 10³ to 1 × 10⁶ cells/cm², more preferably about 1 × 10⁴ to 1 × 10⁵ cells/cm². When the cell is in the above range, the corneal epithelial cell can grow, maintaining its original characteristics.

The culture of the target cell in the step (b) is performed in the absence of heterologous cells. The condition expressed by the phrase "in the absence of heterologous cells" in the present invention means that, for example, when the target cell is a corneal epithelial cell, cells that are heterologous to the corneal epithelial cell are not used in culture of the corneal epithelial cell. In particular, when a human corneal epithelial cell is used as the corneal epithelial cell, this condition means no existence (coexistence) of a cell derived from non-human animal species such as a mouse and a rat in the medium. When the target cell is cultured under such a condition, there will be no risk that a grafting material finally obtained (i.e., a corneal epithelium sheet) is contaminated with heterologous substances (containing a heterologous cell itself).

In the second embodiment of the present invention, human serum may be appropriately added as needed at each step. The added amount and concentration of human serum can be determined according to a conventional method for cell culture and are not specifically limited as long as they do not prevent the effects of the present invention. For example, 5 to 10% human serum can be used.

In the step (b), the medium used for culturing the target cell is not specifically limited as long as the medium supports the growth of the cell concerned. Examples of the medium include MCDB153 medium (NISSUI PHARMACEUTICAL CO., LTD.); EpiLife™ (Cascade Biologics, inc.); a medium produced by modifying the amino acid composition or the like of these media; and a medium produced by mixing DMEM and Ham' s F12 medium, both of which are usually used for growing epithelial cells, at a predetermined ratio. When the target cell is a human corneal epithelial cell, examples of the medium include a DMEM/Ham's F12 mixed medium (mixed volume ratio 1:1) containing FBS (5 to 10%), insulin (for example, 5 µg/ml), cholera toxin (for example, 1 nM), an epidermal growth factor (EGF) (for example, 10 ng/ml), penicillin (for example, 100 U/ml), and streptomycin (for example, 100 µg/ml), but the medium is not specifically limited thereto. The medium that can be used also includes the above DMEM/Ham' s F12 mixed medium further supplemented with glutamine, insulin, transferrin, selenium, or the like. As the conditions such as culture temperature, medium pH and CO₂ concentration, culture conditions commonly used for animal cell culture are employed as appropriate. The culture time is not specifically limited, but is preferably about 2 to 3 weeks.

The production process for the target cell sheet of the present invention may further comprise as needed the step of (c) separating the feeder cell for target cell induction from the target cell sheet. In the step (c), the target cell sheet cultured in the step (b) can be collected by selectively removing the feeder cell with treatment using a precursor of a suicide gene-inducible toxic substance. When an amnion or collagen gel is used as the cell culture carrier, the amnion and collagen gel is not necessarily required to be removed. When a collagen gel is used in the production of the cell sheet of the present invention and the obtained cell sheet is used for transplantation, the cell sheet may be treated with collagenase as needed in view of engraftment with in-vivo cells, but this collagenase treatment may be omitted. The order of carrying out the collagenase treatment and the treatment using a precursor of a suicide gene-inducible toxic substance is not specifically limited. When a temperature-responsive cell culture surface is used as the cell culture carrier, collection of the cell sheet is carried out as follows, after the above antiviral agent treatment: for example, the cell sheet can be collected by changing the temperature of the culture dish from a culture temperature (for example, about 37°C) to a temperature less than the culture temperature (for example, 32°C or less), and leaving the culture dish to stand for about 30 minutes or gently pipetting the medium.

When ganciclovir is used as the precursor of a suicide gene-inducible toxic substance (antiviral agent), the added amount of ganciclovir is not specifically limited because the amount may vary depending on the cell condition after culture, but the concentration of ganciclovir is preferably about 1 × 10⁻² to 1 × 10⁻⁶ M, more preferably about 1 × 10⁻³ to 1 × 10⁻⁵ M. The treatment time is not specifically limited, but is preferably about 4 to 14 days, more preferably about 6 to 10 days. The concentration of collagenase used for the collagenase treatment is not specifically limited because the concentration may vary depending on the cell condition after culture, but the concentration is preferably about 0.01 to 0.5%. The treatment time is preferably about 0.1 to 2 hours, more preferably about 0.5 to 1 hour. The treatment temperature is not specifically limited, but is preferably about 30 to 40°C.

As a result of the step (b), the target cell grows on the cell culture carrier. When the cell to be obtained is a cell that forms multilayers as, for example, corneal epithelial cells do, there may be performed the step of (d) bringing the surface of the cell layer in contact with air for the purpose of promoting formation of multilayers. This step is also called "air-lifting" in this description. The purpose of the step (d) includes differentiation of the cell that forms cell layers and induction of the barrier properties of the cell. The step (d) may be carried out after the step (b), and also may be carried out after the step (c).

This step can be carried out by temporarily removing part of the medium with a pipette dropper, a pipette, or the like so that the medium level is lowered, thereby temporarily making the uppermost surface of the cell layer emerge from the medium surface. Alternatively, this step can be carried out by lifting the cell layer together with a collagen matrix, thereby temporarily making the uppermost surface emerge from the medium surface. Further alternatively, air may be sent into the medium with the use of a tube or the like, thereby bringing the uppermost surface of the cell layer in contact with air. Due to ease of operation, the step is preferably carried out by lowering the medium level, thereby making the uppermost surface of the cell layer emerge from the medium surface.

The duration time of the step (d), i.e., the duration time for making the uppermost surface of the cell layer that formed multilayers in contact with air may vary depending on the cell condition, the culture condition, or the like, but the duration time is, for example, preferably about 3 days to 3 weeks, more preferably about 5 days to 2 weeks.

According to the above production process for a target cell sheet, a cell sheet can be similarly produced from the target cell mentioned above. When the target cell is a stem cell such as a human iPS cell and a human ES cell, the stem cell can be collected as a cell sheet. When various kinds of differentiation inducing factors are introduced, various differentiated cells can be collected as a cell sheet.

Another embodiment of the present invention include, for example,
a process for culturing a human corneal endothelial cell, comprising the steps of
(a) culturing a feeder cell for target cell induction, obtained as described above, on a cell culture carrier,
(b) culturing and growing a human corneal endothelial cell by seeding the human corneal endothelial cell on the cell culture carrier of the step (a) and then by allowing the human corneal endothelial cell to stand;
a process for culturing a human conjunctival epithelial cell, comprising the steps of
(a) culturing a human corneal stromal cell line, obtained as described above, on a cell culture carrier,
(b) culturing a human conjunctival epithelial cell prepared in the above step (b) by seeding the human conjunctival epithelial cell on the cell culture carrier of the step (a) and then by allowing the human conjunctival epithelial cell to stand; and
a process for culturing a human kidney-derived cell, comprising the steps of
(a) culturing a feeder cell for target cell induction, obtained as described above, on a cell culture carrier,
(b) culturing a human kidney-derived cell prepared in the above step (b) by seeding the human kidney-derived cell on the cell culture carrier of the step (a) and then by allowing the human kidney-derived cell to stand.

According to the culture process using the feeder cell of the present invention, it is possible to determine, as appropriate, the culture cell density of an intended target cell depending on the kind of the target cell. For example, even when culturing a target cell at a high density is not required and the target cell is cultured at a low density, the target cell having more similar conditions to those in a living body can be obtained. The cell density is not specifically limited because the cell density may be adjusted as appropriate depending on the kind of the target cell, and the cell density of the target cell may be, for example, about 0.5 × 10¹ to 9 × 10² cells/cm².

Another embodiment of the present invention includes a process for determining a candidate inducing factor for a target cell, comprising the steps of
(a') culturing a feeder cell for target cell induction of the present invention on a cell culture carrier,
(b') transducing a feeder cell of the present invention with the gene of a candidate inducing factor for the target cell, and culturing the candidate inducing factor-transduced feeder cell on a cell culture carrier,
(c') culturing and growing the target cell by seeding the target cell on each of the cell culture carriers of the above (a') and (b') and then by allowing the target cell to stand, and
(d') evaluating the cells cultured on each of the cell culture carriers.

The candidate inducing factor is not specifically limited as long as it does not prevent the effects of the present invention, and the candidate inducing factor may be, for example, the above stimulating factor for a target cell. The culture process of the cell can be carried in the same manner as in the above process. The target cell and the cell culture carrier may be the same as those described above. In the step of evaluating the cells, the evaluation method can be appropriately selected from conventional methods depending on the kind of the target cell. For example, by counting the number of colony formation, the effect on cell growth can be evaluated. Thus, according to the present invention, the function of a candidate inducing factor for a target cell can be evaluated and determined, and thereby the stimulating factor for a target cell can be screened.

Another embodiment of the present invention includes a process for culturing a target cell, comprising the steps of
(i) transducing a feeder cell of the present invention with the gene of a candidate inducing factor for the target cell,
(ii) culturing the candidate inducing factor-transduced feeder cell obtained in the step (i) on a cell culture carrier, and
(iii) culturing and growing the target cell by seeding the target cell on the cell culture carrier of the step (ii) and then by allowing the target cell to stand.

The candidate inducing factor is not specifically limited as long as it does not prevent the effects of the present invention, and the candidate inducing factor may be, for example, the above stimulating factor for a target cell. The culture process of the cell can be carried in the same manner as in the above process. The target cell and the cell culture carrier may be the same as those described above.

Another embodiment of the present invention further include a process for transplanting a target cell, comprising the step of transplanting a target cell sheet, obtained by the above production process for a target cell sheet, into an intended site (for example, human corneal surface, but not specifically limited thereto).

### EXAMPLES

The present invention will be explained more specifically with reference to the following Examples, but the present invention is not limited thereto.

### EXAMPLE 1

A human corneal stromal cell line transduced with the hTERT gene and the TK gene (hereinafter referred to as hTERT+TK-transduced human corneal stromal cells) was produced by the following procedure. The schematic view of the production of the hTERT+TK-transduced human corneal stromal cells is shown in Fig. 2.

### 1. Culture of Human Corneal Stromal Cells

Epithelial cells and endothelial cells were removed (scraped off with forceps) from a piece of corneoscleral tissue obtained from an imported cornea (USA Eye Bank). The corneal stroma thus obtained was cut into several pieces and cultured with its endothelial side down in a 35-mm dish containing DMEM, 10% FBS, 100 U/ml penicillin and 100 µg/ml streptomycin under conditions of 37°C and 5% CO₂.

### 2. Preparation of Lentiviral Vectors

Transduced lentiviral vectors were produced by the following procedure using a packaging plasmid and a gene-of-interest expression vector that is required for the production of the lentivirus shown in Fig. 1.

### (1) Production of hTERT-, GFP- and Neomycin-Resistance Gene Expression Lentiviral Vector

The hTERT gene fragment shown in SEQ ID: NO.1 and an IRES-EGFP gene fragment were subcloned downstream of the EF1 promoter that lies between the long terminal repeats (LTRs) of a lentiviral vector. Further downstream, a neomycin-resistance gene was placed downstream of the PGK promoter. This lentiviral vector is shown in Fig. 1A.

### (2) Production of TK- and Puromycin-Resistance Gene Expression Lentiviral Vector

The TK gene fragment shown in SEQ ID: NO. 3 was subcloned downstream of the EF1 promoter in a lentiviral vector using the primers shown in SEQ ID: NOs. 5 and 6. Further downstream, a puromycin-resistance gene was placed downstream of the PGK promoter. This lentiviral vector is shown in Fig. 1B.

### (3) Production of hTERT-, GFP-, and Neomycin-Resistance Gene Expression Lentivirus (Recombinant hTERT Lentivirus), and TK- and Puromycin-Resistance Gene Expression Lentivirus (Recombinant TK Lentivirus)

About 2 × 10⁶ cells of 293T cells were seeded in a 10-cm dish and the cells were cultured under conditions of 37°C and 5% CO₂ (medium: DMEM, 10% FBS, 100 U/ml penicillin and 100 µg/ml streptomycin). One to two days after culture (50 to 80% confluency), the medium was replaced about 3 hours prior to transfection, and the cells were transfected with the hTERT-, GFP-, and neomycin-resistance gene expression lentiviral vector plasmid produced as described above and with a packaging plasmid (pLP1 (gag/pol), a REV plasmid (pLP2 (Rev)) and an envelope plasmid (pLP/VSVG (VSV-G)) using a transfection kit (trade name: CalPhos™ Mammalian Transfection Kit (Clontech Laboratories, Inc.)). One day after transfection, the medium was replaced. One day after replacement of the medium, the culture supernatant was collected (by filtration using a 0.45 µm filter) and ultracentrifuged (at 4°C and 20000 rpm for 2 hours). The sediment was suspended in a DMEM medium, and thus an hTERT-, GFP- and neomycin-resistance gene expression lentivirus (hereinafter referred to as recombinant hTERT lentivirus") was prepared (stored at -80°C).

Similarly, a TK- and puromycin-resistance gene expression lentivirus (hereinafter referred to as "recombinant TK lentivirus") was prepared (stored at -80°C) using the TK- and puromycin-resistance gene expression lentiviral vector.

### 3. Production of hTERT+TK-transduced Human Corneal Stromal Cells (Feeder cells)

The human corneal stromal cells cultured as described above were infected with the above recombinant hTERT lentivirus. From the 3rd day after infection, selection was carried out with G418 (800 µg/ml), and thus hTERT-transduced human corneal stromal cells were obtained. The hTERT-transduced human corneal stromal cells were further infected with the above recombinant TK lentivirus. From the 3rd day after infection, selection was carried out with 1 µg/ml puromycin, and thus hTERT+TK-transduced human corneal stromal cells were obtained. The schematic view of the production process is shown in Fig. 2.

### 4. Results

In the hTERT+TK-transduced human corneal stromal cells, the expression (fluorescence) of the marker protein EGFP confirmed the introduction of the hTERT gene. The human corneal stromal cells prior to hTERT-transduction were able to be maintained only for up to about 9 passages under conditions of 37°C and 5% CO₂ (medium: DMEM, 10% FBS, 100 U/ml penicillin and 100 µg/ml streptomycin). On the other hand, the hTERT+TK-transduced human corneal stromal cells, which had transduced with the hTERT gene, were able to be maintained for as long as 50 passages or more.

### COMPARATIVE EXAMPLE

hTERT-transduced human corneal stromal cells were produced in the same manner as in Example 1 except that the cells were not transduced with the TK gene.

### TEST EXAMPLE

The susceptibilities of the transduced human corneal stromal cells to ganciclovir treatment were investigated by the following process. The hTERT-transduced human corneal stromal cells of Comparative Example and the hTERT+TK-transduced human corneal stromal cells of Example 1 were separately seeded at 2 x 10⁴ cells/cm² in a 24-well plate, and cultured under conditions of 37°C and 5% CO₂ (medium: DMEM, 10% FBS, 100 U/ml penicillin and 100 µg/ml streptomycin). On the 1st day after seeding, ganciclovir treatment was started (treatment concentration: 0, 10⁻⁷, 10⁻⁶, 10⁻⁵ and 10⁻⁴ M; time course: day 0, 2, 4, 6, 8, 10, and 12). The susceptibility of each type of the transduced human corneal stromal cells to ganciclovir treatment was determined using 3 wells for each concentration at each time point (n = 3). At each time point, the number of the cells was counted with trypan-blue staining. For counting with trypan-blue staining, an erythrocytometer (hematometer; product name: Burker-Turk deep 1/10 mm; product number: Tokyo No. 0880 (ERMA, Inc.)) was used. Immediately after staining, each sample was placed in a counting chamber of the erythrocytometer. Immediately after that, the number of the cells was counted. The counting results are shown in Fig. 3. The average number of the cells on the zero-day of ganciclovir treatment at each treatment concentration was used as a baseline (100%) for the results of the cell number at each time point.

As shown in Fig. 3, the number of the hTERT-transduced human corneal stromal cells of Comparative Example did not decrease at any treatment concentration on the 12th day after ganciclovir treatment (Fig. 3, left). On the other hand, in the 10⁻⁴ M treatment group of the hTERT+TK-transduced human corneal stromal cells, all the cells died on the 6th day after ganciclovir treatment (Fig. 3, right). Moreover, in other treatment concentration groups (10⁻⁷, 10⁻⁶ , and 10⁻⁵ M), a concentration-dependent decrease in the number of the cells was observed from the 8th day after ganciclovir treatment.

### EXAMPLE 2

A human corneal epithelial cell sheet was produced by the following procedure. The schematic view of the production process for the human corneal epithelial cell sheet is shown in Fig. 4A.

### 1. Culture of hTERT+TK-transduced Human Corneal Stromal Cells on Collagen Gel

A collagen gel (type I collagen) was produced in a 6-well plate using Collagen Gel Culture Kit (Nitta Gelatin). On this collagen gel (Nitta Gelatin), the hTERT+TK-transduced human corneal stromal cells produced in Example 1 were seeded, and cultured under conditions of 37°C and 5% CO₂ (medium: DMEM, 10% FBS, 100 U/ml penicillin and 100 µg/ml streptomycin). The photograph of the hTERT+TK-transduced human corneal stromal cells (feeder cells) obtained by culture is shown in Fig. 4B (top).

### 2. Preparation of Human Corneal Epithelial Cells

A piece of corneoscleral tissue obtained from an imported cornea (USA Eye Bank) was incubated in a 2.4 U/ml Dispase solution at 37°C for 1 hour. Next, the tissue was treated with a 0.02% EDTA solution at room temperature for 2 minutes and washed twice with PBS. The corneal epithelial stem cells around the corneal limbus were scraped off from the obtained cell mass with forceps under a microscope and under immersion in a corneal epithelial cell culture medium (DMEM/F-12, 5% FBS, insulin-transferrin-selenium, 1 nM cholera toxin, 10 ng/ml EGF, 100 U/ml penicillin and 100 µg/ml streptomycin). The obtained corneal epithelial stem cells were centrifuged at 1000 rpm for 5 minutes. After removal of the supernatant, 1 ml of a 0.25% trypsin-EDTA solution was added to the cells, and the cell suspension was incubated at 37°C for 15 to 20 minutes. Next, the equivalent amount (1 ml) of a corneal epithelial cell culture medium was added to the cells, and the cell suspension was centrifuged at 1000 rpm for 5 minutes. After removal of the supernatant, the cells were suspended in a corneal epithelial cell culture medium to give a suspension of the human corneal epithelial cells.

### 3. Seeding and Culturing of Human Corneal Epithelial Cells

The above suspension was seeded on the hTERT+TK-transduced human corneal stromal cells cultured on the collagen gel so that the cell density of the human corneal epithelial cells was about 6 × 10⁴ cells/cm², and the cells were cultured under conditions of 37°C and 5% CO₂ (medium: DMEM/F-12, 5% FBS, insulin-transferrin-selenium, 1 nM cholera toxin, 10 ng/ml EGF, 100 U/ml penicillin and 100 µg/ml streptomycin). On the 3rd or 4th day after seeding, the medium was replaced and the cells were cultured for 18 days. The photograph of the human corneal epithelial cells obtained by culture is shown in Fig. 4B (middle).

### 4. Antiviral Agent Treatment and Collagenase Treatment

After the above culture, the cells were cultured with the addition of 10⁻⁴ M ganciclovir for about 1 week (the medium was replaced once every 2 or 3 days) so that the hTERT+TK-transduced human corneal stromal cells were completely killed. Next, a collagenase solution (0.1%) was added, and the cells were incubated at 37°C for about 30 minutes, and thereby a corneal epithelial cell sheet was collected. However, the collagenase treatment is not necessarily required. The photograph of the obtained corneal epithelial cell sheet is shown in Fig. 4B (bottom).

### EXAMPLE 3

A corneal epithelial cell sheet was collected in the same manner as in "Culture of hTERT+TK-transduced Human Corneal Stromal Cells on Collagen Gel" of Example 2 except that the hTERT+TK-transduced human corneal stromal cells were treated with mitomycin C (8 µg/ml) at 37°C for 2 hours before the cells were seeded on the collagen gel.

From Examples 2 and 3, it was confirmed that a human corneal epithelial cell sheet can be produced using the feeder cells of the present invention. Since the production process of the present invention does not require the use of mouse 3T3 cells, which are commonly used as feeder cells, the risk of infection from heterologous cells is avoidable. In addition, while human corneal stromal cells obtained from primary culture can be maintained only for several passages and thus have limitations on use for transplantation, the feeder cell line of the present invention can be maintained through long-term passage as a result of introduction of the TERT gene therein. Further, since a suicide gene (TK) and a marker gene have been introduced into the feeder cells in view of the risk of contamination with the feeder cells, the feeder cells can be killed by ganciclovir when they become unnecessary, and contamination with the feeder cells can be visually observed. Since the feeder cells can be killed by ganciclovir, adherent culture with corneal epithelial cells is also possible. Moreover, since ganciclovir is clinically available as an anti-herpesvirus agent, even when the cell sheet has been contaminated with the feeder cells in its production and then the cell sheet has been transplanted, the feeder cells can be killed and removed by administration of ganciclovir.

### EXAMPLE 4

### 1. Culture of hTERT+TK-transduced Human Corneal Stromal Cells on Collagen Gel

hTERT+TK-transduced human corneal stromal cells were cultured on a collagen gel in the same manner as in "Culture of hTERT+TK-transduced Human Corneal Stromal Cells on Collagen Gel" of Example 2.

### 2. Preparation of Human Conjunctival Epithelial Cells

Human conjunctival epithelial cells were prepared in the same manner as in "Preparation of Human Corneal Epithelial Cells" of Example 2 except that the procedure after washing twice with PBS was replaced with the following procedure.

After the obtained cell mass was washed with PBS, only a conjunctiva was excised therefrom. The obtained conjunctiva was cut into pieces with scissors under immersion in a conjunctival epithelial cell culture medium (DMEM/F-12, 5% FBS, insulin-transferrin-selenium, 1 nM cholera toxin, 10 ng/ml EGF, 100 U/ml penicillin, and 100 µg/ml streptomycin). The pieces of the cells were centrifuged at 1000 rpm for 5 minutes. After removal of the supernatant, 1 ml of a 0.25% trypsin-EDTA solution was added to the cells, and the cell suspension was incubated at 37°C for 15 to 20 minutes. Next, the equivalent amount (1 ml) of a conjunctival epithelial cell culture medium was added to the cells. After cell lumps were removed with a filter, the cell suspension was centrifuged at 1000 rpm for 5 minutes. After removal of the supernatant, the cells were suspended in a conjunctival epithelial cell culture medium to give a suspension of the human conjunctival epithelial cells.

### 3. Seeding and Culturing of Human Conjunctival Epithelial Cells

The above suspension was seeded on the hTERT+TK-transduced human corneal stromal cells cultured on the collagen gel so that the cell density of the human conjunctival epithelial cells was about 4 x 10⁴ cells/cm², and the cells were cultured under conditions of 37°C and 5% CO₂ (medium: DMEM/F-12, 5% FBS, insulin-transferrin-selenium, 1 nM cholera toxin, 10 ng/ml EGF, 100 U/ml penicillin and 100 µg/ml streptomycin). On the 3rd or 4th day after seeding, the medium was replaced and the cells were cultured for 18 days.

From the above, it was confirmed that human conjunctival epithelial cells can be cultured on the hTERT+TK-transduced human corneal stromal cells as feeder cells. The photograph of the cells obtained by culture is shown in Fig. 5A.

### EXAMPLE 5

hTERT+TK-transduced human corneal stromal cells were cultured on a collagen gel in the same manner as in "Culture of hTERT+TK-transduced Human Corneal Stromal Cells on Collagen Gel" of Example 2. Next, immortalized human corneal endothelial cells were seeded on the hTERT+TK-transduced human corneal stromal cells, and cultured under conditions of 37°C and 10% CO₂ (medium: DMEM, 10% FBS, 30 µg/ml L-glutamine, 2 ng/ml b-FGF, 100 U/ml penicillin and 100 µg/ml streptomycin).

From the above, it was confirmed that human corneal endothelial cells can be cultured on the hTERT+TK-transduced human corneal stromal cells as feeder cells. The photograph of the cells obtained by culture is shown in Fig. 5B.

### EXAMPLE 6

hTERT-TK-transduced human corneal stromal cells were cultured on a collagen gel in the same manner as in "Culture of hTERT+TK-transduced Human Corneal Stromal Cells on Collagen Gel" of Example 2. Human kidney-derived cells (293T cells) were seeded on the TERT+TK-transduced human corneal stromal cells, and cultured under conditions of 37°C and 5% CO₂ (medium: DMEM, 10% FBS, 100 U/ml penicillin and 100 µg/ml streptomycin).

From the above, it was confirmed that human kidney-derived cells can be cultured on the hTERT+TK-transduced human corneal stromal cells as feeder cells. The photograph of the cells obtained by culture is shown in Fig. 5C.

From the results of Examples 4 to 6, it was confirmed that the feeder cells of the present invention are applicable to cell culture of not only corneal epithelial cells but also various kinds of cells such as conjunctival epithelial cells, corneal endothelial cells, and kidney-derived cells.

### EXAMPLE 7

Human corneal epithelial cells were cultured in the same manner as in culture of the hTERT+TK-transduced human corneal stromal cells in Example 1 (medium: DMEM, 10% human serum, 100 U/ml penicillin and 100 µg/ml streptomycin) and as in culture of human corneal epithelial cells in Example 2 (medium:
DMEM/F-12, 5% human serum, insulin-transferrin-selenium, 1 nM cholera toxin, 10 ng/ml EGF, 100 U/ml penicillin and 100 µg/ml streptomycin) except that, instead of FBS, human serum (No. 14-402E (Lonza)) was used for the medium. As a result, the human corneal epithelial cells were able to be cultured. The photograph of the human corneal epithelial cells obtained by culture using human serum is shown in Fig. 6.

### EXAMPLE 8

A human corneal epithelial cell sheet was transplanted in a male ICR mouse (SLC). The schematic view of the transplantation procedure is shown in Fig. 7A. The transplantation was carried out under intraperitoneal anesthesia with Nembutal. The corneal surface including the limbus was exposed to 50% ethanol for about 30 seconds, and superficial keratectomy was performed. That is, immediately after the cell layer of the corneal epithelium was completely removed by exposure to 50% ethanol, a human corneal epithelial cell sheet that had been produced in the same manner as in Example 2 (without collagenase treatment) and cut out in a 5-mm diameter circle with a trephine was transplanted onto the corneal surface. The cell sheet and the eyelid were sutured with 8.0 sutures for surgery. The effect of the transplantation was evaluated in comparison with a control mouse onto which a human corneal epithelial cell sheet was not transplanted. After transplantation, antibiotics and steroids were administered. Seven days after transplantation, the eyeball was removed and fixed with 4% paraformaldehyde at 4°C for 3 to 4 hours. The results are shown in Fig. 7B (control) and Fig. 7C (transplanted specimen). The specimens were prepared by Technovit embedding (Technovit 8100 (Heraeus Kluzer)), and 5-µm sections were prepared. The sections were stained with HE (hematoxylin-eosin) and immunostained. The HE staining was carried out according to a conventional method, and the result is shown in Fig. 7D. Immunostaining was carried out by the following procedure. The sections were treated with 0.05% trypsin-EDTA at 37°C for 10 minutes, and washed with PBS. The sections were treated with a blocking solution (Block Ace (DS pharma biomedical) and 0.3% tritonX-100) at room temperature for 1 hour, and reacted with a primary antibody at 4°C overnight (primary antibody: a mouse monoclonal anti-cytokeratin 3 antibody (1:50 (Progen)), or a mouse monoclonal anti-human mitochondria antibody (1:10 (Millipore))). After washed with PBS, the sections were reacted with a secondary antibody at room temperature for 60 minutes (second antibody: Alexa Fluor 488 anti-mouse IgG antibody (1:200 (Invitrogen))). After the sections were washed with PBS, nuclear staining (0.1 µg/ml DAPI (Sigma)) was carried out at room temperature for 5 minutes. After washed with PBS, the sections were sealed with a water soluble mounting medium.

As is apparent from Fig. 7B, in the specimen of the mouse onto which a human corneal epithelial cell sheet was not transplanted (control), the cornea was completely covered with conjunctival tissue containing blood vessels and the transparency of the cornea was lost on the 15th day after the treatment. On the other hand, as shown in Fig. 7C, on the eye on which transplantation was performed, invasion by conjunctival tissue was not observed and the cornea remained transparent on the 7th day after the transplantation. From the result of microscopic observation as shown in Fig. 7D, it was observed that the corneal epithelial cells formed 3 to 5 layers. From the result of immunostaining as shown in Fig. 7F, the expression of cytokeratin 3, a marker for corneal epithelial cells, was observed. For the purpose of verifying that the corneal epithelial cells shown in Figs. 7D and 7F were the human-derived transplanted cells, immunostaining for human mitochondria was performed in the same manner as in the above. The result is shown in Fig. 7E. This result shows that the corneal epithelial cells were human-derived cells. Overall, the above results show that the human corneal epithelial cell sheet produced using the hTERT+TK-transduced human corneal stromal cells can be implanted in a mouse model of corneal epithelial damage and can be compatible with a living body.

### EXAMPLE 9

According to the following procedure, a candidate inducing factor for a target cell was introduced, using the lentivirus below, into the hTERT+TK-transduced human corneal stromal cells obtained in Example 1 to give cells expressing an inducing factor.

### 1. Preparation of Lentiviral Vectors

Transduced lentiviral vectors were produced by the following procedure using a packaging plasmid and a gene-of-interest expression vector that is required for the production of the lentivirus shown in Fig. 8.

### (1) Production of DLL1/DLL4- and Blasticidin-Resistance Gene Expression Lentiviral Vector

A DLL1/DLL4- and blasticidin-resistance gene expression lentiviral vector was produced by subcloning a Delta-like 1 (DLL1) gene fragment or a Delta-like 4 (DLL4) gene fragment downstream of the UbC promoter in a lentiviral vector (pLenti6/UbC/V5-DEST (Invitrogen)). This lentiviral vector is shown in Fig. 8A.

### (2) Production of E-cadherin- and Hygromycin-Resistance Gene Expression Lentiviral Vector

An E-cadherin gene fragment was subcloned downstream of the EF1 promoter in a lentiviral vector. Further downstream, a hygromycin-resistance gene was placed downstream of the PGK promoter. Thus an E-cadherin- and hygromycin-resistance gene expression lentiviral vector was produced. This lentiviral vector is shown in Fig. 8B.

### (3) Production of DLL1- and Blasticidin-Resistance Gene Expression Lentivirus (Recombinant DLL1 Lentivirus), DLL4- and Blasticidin-Resistance Gene Expression Lentivirus (Recombinant DLL4 Lentivirus), and E-cadherin- and Hygromycin-Resistance Gene Expression Lentivirus (Recombinant E-cadherin Lentivirus)

In the same manner as in the production process for the above lentiviruses, a DLL1- and blasticidin-resistance gene expression lentivirus (hereinafter referred to as "recombinant DLL1 lentivirus"), a DLL4- and blasticidin-resistance gene expression lentivirus (hereinafter referred to as "recombinant DLL4 lentivirus"), and an E-cadherin- and hygromycin-resistance gene expression lentivirus (hereinafter referred to as "recombinant E-cadherin lentivirus") were prepared (stored at -80°C).

### 2. Production of DLL1+, DLL4+, or E-cadherin+, hTERT+TK-transduced Human Corneal Stromal Cells

The recombinant DLL1 lentivirus, the recombinant DLL4 lentivirus, and the recombinant E-cadherin lentivirus were separately used to infect the hTERT+TK-transduced human corneal stromal cells obtained in Example 1. From the 2nd day after infection, selection was carried out with blasticidin (10 µg/mL) or hygromycin (50 µg/ml), and thus DLL1+hTERT+TK-transduced human corneal stromal cells, DLL4+hTERT+TK-transduced human corneal stromal cells, and E-cadherin+hTERT+TK-transduced human corneal stromal cells were produced. RNA was extracted from each of the hTERT+TK-transduced human corneal stromal cell, the DLL1+hTERT+TK-transduced human corneal stromal cell, the DLL4+hTERT+TK-transduced human corneal stromal cell, and E-cadherin+hTERT+TK-transduced human corneal stromal cell (RNeasy Mini Kit (Qiagen)). After each cell was treated with DNase I (Invitrogen), the cDNA of each cell was synthesized (First-strand cDNA Synthesis System for Quantitative RT-PCR (Marligen biosciences)). PCR reaction was performed using the prepared cDNA. The PCR reaction conditions are shown below. PCR primers were designed for each of DLL1 (470 bp), DLL4 (249 bp), E-cadherin (172 bp), and, as a control, GAPDH (255 bp). The primers used are shown in SEQ ID: NOs. 7 to 14. PCR reaction products were electrophoresed on a 2% agarose gel.

### «PCR conditions »

94°C for 5 min; 35 cycles of 94°C for 30 sec, 60°C for 30 sec, and 72°C for 30 sec; and 72°C for 10 min

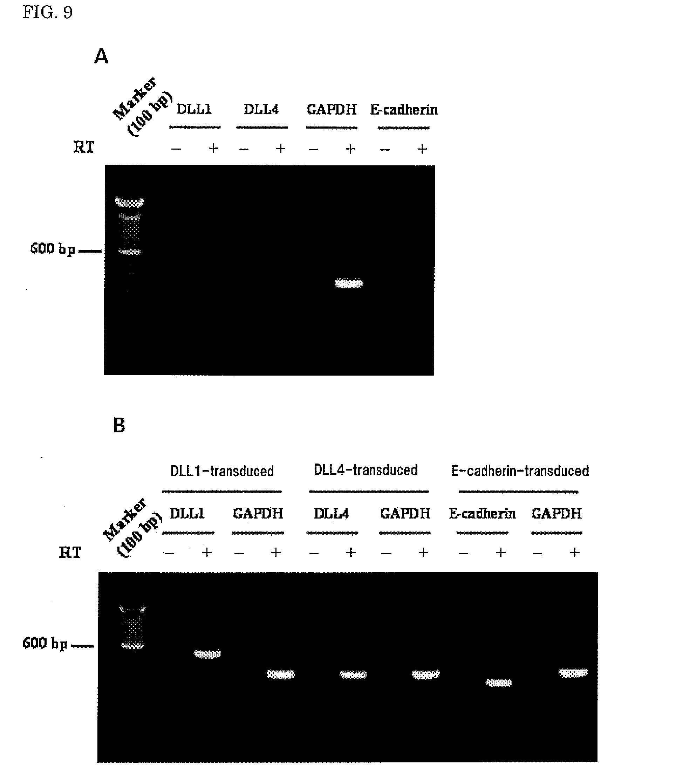

From the results of the RT-PCR, expressions of DLL1, DLL4, and E-cadherin were not observed for the hTERT+TK--transduced human corneal stromal cells (Fig. 9A). On the other hand, expression of each gene of DLL1, DLL4, and E-cadherin was observed for the cells infected with the corresponding recombinant lentivirus (Fig. 9B). From the above results, it was confirmed that a inducing factor for target cell induction can be further introduced into the hTERT+TK-transduced human corneal stromal cells. That is, introduction of various kinds of inducing factors into the transduced human corneal stromal cells of the present invention enables the cells to serve as feeder cells for inducing a target cell which is difficult to culture or of which differentiation is difficult to induce.

### EXAMPLE 10

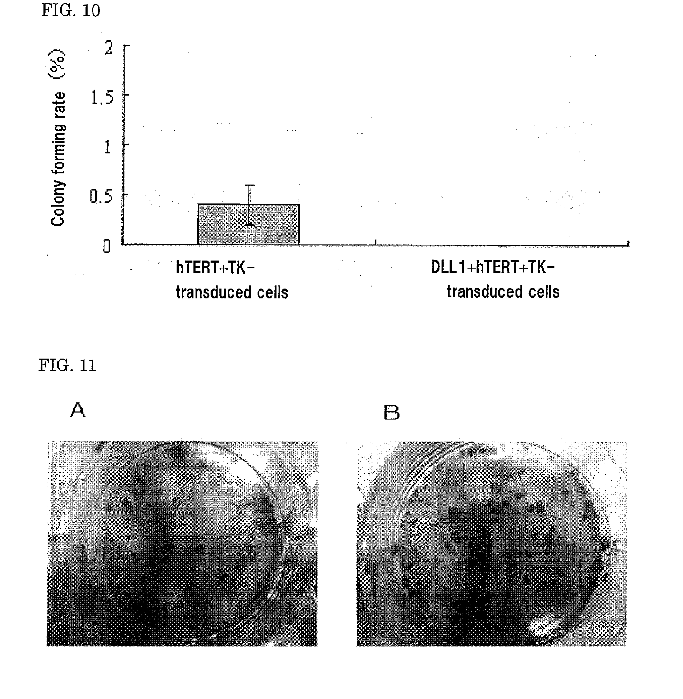

Human corneal epithelial cells were cultured on the feeder cell described below in the same manner as in "Preparation of Human Corneal Epithelial Cells" and "Seeding and Culturing of Human Corneal Epithelial Cells" of Example 2. The hTERT+TK--transduced human corneal stromal cells obtained in Example 1 and the DLL1+hTERT+TK-transduced human corneal stromal cells obtained in Example 9 were separately used as feeder cells, and 1000 cells of human corneal epithelial cells were seeded on each kind of the feeder cells (3 wells each). On the 12th day after seeding, the cells were fixed with 10% formaldehyde at room temperature for 30 minutes. The cells were stained with a 1% rhodamine solution (Wako) at room temperature for 10 minutes and washed with PBS. The number of the stained colonies was visually counted. The counting results of the colonies are shown in Fig. 10.

As shown in Fig. 10, when the DLL1+hTERT+TK-transduced human corneal stromal cells were used as feeder cells (right), colony formation was inhibited as compared with the case where the hTERT+TK-transduced human corneal stromal cells were used as feeder cells (left). This result revealed that DLL1 has the function of inhibiting colony formation of corneal epithelial cells. As described above, by introducing various kinds of factors into the transduced human corneal stromal cells of the present invention, the function of the introduced factor on a target cell can be evaluated.

### EXAMPLE 11

Human dermal fibroblasts were transduced with the hTERT+TK gene by the following procedure. In addition, colony formation of human corneal epithelial cells was observed.

### 1. Production of hTERT+TK-transduced Human Dermal Fibroblasts (Feeder Cells)

Normal human dermal fibroblasts (Kurabo Industries Ltd.; product number: KF-4109) were infected with the recombinant hTERT lentivirus and the recombinant TK lentivirus, both of which were used in Example 1. From the 2nd day after infection, selection was carried out with G418 (800 µg/ml) and puromycin (1 µg/ml), and thus hTERT+TK-transduced human dermal fibroblasts were produced (medium: low-serum normal human dermal fibroblast growth medium (Medium 106S; Kurabo Industries Ltd.; product number: M-106-500S), low-serum growth supplement (LSGS; Kurabo Industries Ltd.; product number:S-003-5), 100 U/ml penicillin and 100 µg/ml streptomycin).

### 2. Preparation of Human Corneal Epithelial Cells and Seeding and Culturing of Human Corneal Epithelial Cells

hTERT+TK-transduced human dermal fibroblasts obtained as described above and the hTERT+TK-transduced human corneal stromal cells of Example 1 were separately used as feeder cells, and human corneal epithelial cells were cultured on each kind of the feeder cells in the same manner as in "Preparation of Human Corneal Epithelial Cells" and "Seeding and Culturing of Human Corneal Epithelial Cells" of Example 2. 1000 cells of human corneal epithelial cells were seeded on each kind of the feeder cells. On the 10th day after seeding, the cells were fixed with 10% formaldehyde at room temperature for 30 minutes. The cells were stained with a 1% rhodamine solution (Wako) at room temperature for 10 minutes and washed with PBS. The result is shown in Fig. 11.

### 3. Results

The result in Fig. 11B shows that when human corneal epithelial cells were cultured on the hTERT+TK-transduced human dermal fibroblasts as feeder cells, the human corneal epithelial cells formed colonies in the same manner as the case where the hTERT+TK-transduced human corneal stromal cells were used as feeder cells (Fig. 11A). This result revealed that the hTERT+TK-transduced human dermal fibroblasts can be used to culture corneal epithelial cells. From the above results, it was confirmed that even different kinds of cell strains can be used as feeder cells by introducing the hTERT gene and the TK gene. That is, introduction of the hTERT+TK gene into an optimal feeder cell for culturing a target cell enables target cell induction and production of a target cell sheet.

### EXAMPLE 12

### 1. Culture of Human Corneal Endothelial Cells on hTERT+TK-transduced Human Corneal Stromal Cells

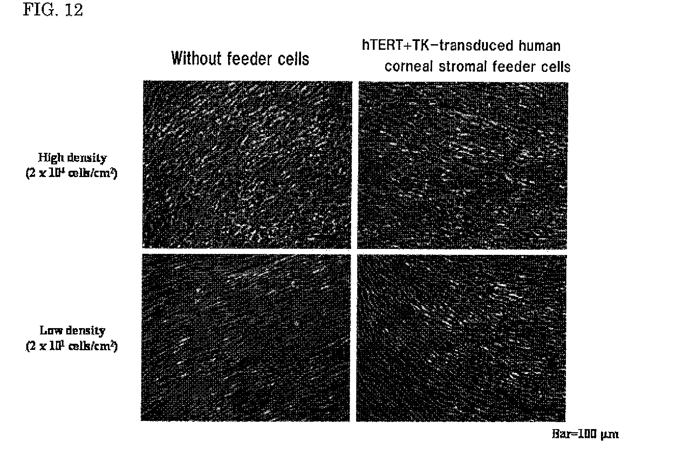

Corneal endothelial cells including Descemet membrane were harvested from a piece of corneoscleral tissue obtained from an imported cornea (USA Eye Bank) using forceps under a stereoscopic microscope, and the cells were cultured with its corneal endothelial side down in a 6-well plate coated with FNC Coating Mix (Athena Enzyme Systems) under conditions of 37°C and 10% CO₂ (medium: DMEM, 10% FBS, 25 ng/ml b-FGF, 100 U/ml penicillin and 100 µg/ml streptomycin). Next, 2 × 10⁴ cells/cm² of hTERT+TK-transduced human corneal stromal cells treated with mitomycin C (8 µg/ml) were seeded in a 6-well plate, and cultured under conditions of 37°C and 5% CO₂ (medium: DMEM, 10% FBS, 100 U/ml penicillin and 100 µg/ml streptomycin). On the 1st day after seeding, the human corneal endothelial cells at high density (2 × 10⁴ cells/cm²) and the human corneal endothelial cells at low density (2 × 10¹ cells/cm²) were separately seeded on the hTERT+TK-transduced human corneal stromal cells, and cultured under conditions of 37°C and 10% CO₂ (medium: DMEM, 10% FBS, 25 ng/ml b-FGF, 100 U/ml penicillin and 100 µg/ml streptomycin). As a control, the human corneal endothelial cells at high density (2 × 10⁴ cells/cm²) and the human corneal endothelial cells at low density (2 × 10¹ cells/cm²) were separately seeded in a 6-well plate coated with FNC Coating Mix (without feeder cells), and the cells were cultured. On the 26th day after seeding, the cells were observed. The observation results are shown in Fig. 12.

### 2. Results

When the corneal endothelial cells were cultured at high density, independently of the presence or absence of the feeder cells, the corneal endothelial cells were, during culture, able to maintain similar cell forms to those of corneal endothelial cells in a living body, which cell form is hexagon (Fig. 12, top left and top right). When the corneal endothelial cells were cultured at low density on the feeder cells, the corneal endothelial cells had similar cell forms to those of the corneal endothelial cells cultured at high density (Fig. 12, bottom right); however, in the absence of the feeder cells, the corneal endothelial cells at low density had similar cell forms to those of fibroblasts (Fig. 12, bottom left). From the above results, by culturing human corneal endothelial cells on hTERT+TK-transduced human corneal stromal cells as feeder cells, human corneal endothelial cells having similar conditions to those of corneal endothelial cells in a living body can be quickly obtained.

### INDUSTRIAL APPLICABILITY

The feeder cell for target cell induction of the present invention is useful for cell culture and production of a cell sheet. In particular, the feeder cell is useful for production of a human corneal epithelial cell sheet that can be used for transplantation intended for corneal regeneration.

## Claims

1. A feeder cell for target cell induction, wherein the feeder cell is transduced with an immortalizing gene and a suicide gene.

2. The feeder cell for target cell induction according to claim 1, wherein the feeder cell is derived from a cell that is contiguous to the target cell.

3. The feeder cell for target cell induction according to claim 1, wherein the feeder cell is derived from a human corneal stromal cell.

4. The feeder cell for target cell induction according to claim 1, wherein the feeder cell is derived from a human dermal fibroblast.

5. The feeder cell for target cell induction according to claim 1, wherein the immortalizing gene is an hTERT gene.

6. The feeder cell for target cell induction according to claim 1 or 4, wherein the suicide gene is herpes simplex virus type 1 thymidine kinase gene.

7. The feeder cell for target cell induction according to claim 1, wherein the feeder cell is further transduced with a marker gene.

8. The feeder cell for target cell induction according to claim 7, wherein the marker gene is an EGFP gene.

9. The feeder cell for target cell induction according to claim 1, wherein the target cell is a human-derived cell that requires a feeder cell for cell culture.

10. The feeder cell for target cell induction according to claim 1, wherein the target cell is a human corneal epithelial cell, a human corneal endothelial cell, a human conjunctival epithelial cell, or a human kidney-derived cell.

11. The feeder cell for target cell induction according to claim 1, wherein the feeder cell is further transduced with the gene of a stimulating factor for the target cell.

12. The feeder cell for target cell induction according to claim 11, wherein the stimulating factor for the target cell is one or more human cell adhesion molecules selected from a cadherin superfamily, an integrin family, an immunoglobulin superfamily, a secretin family, a neuroligin, a neurexin, and a cell surface proteoglycan; one or more growth factors selected from an epidermal growth factor (EGF) family, a vascular endothelial cell growth factor (VEGF) family, an insulin-like growth factor (IGF) family, and a fibroblast growth factor (FGF) family; or one or more Notch ligands selected from a Jagged family and a Delta-like member.

13. A production process for a target cell sheet, comprising the steps of
(a) culturing a feeder cell for target cell induction according to claim 1 on a cell culture carrier, and
(b) culturing and growing the target cell by seeding the target cell on the cell culture carrier of the step (a) and then by allowing the target cell to stand.

14. The production process according to claim 13, wherein the process further comprises the step of (c) separating the feeder cell for target cell induction from the target cell sheet.

15. The production process according to claim 14, wherein the separation in the step (c) is carried out by treating the target cell sheet with a precursor of a suicide gene-inducible toxic substance.

16. The production process according to claim 15, wherein the suicide gene is herpes simplex virus type 1 thymidine kinase gene, and the precursor of a suicide gene-inducible toxic substance is ganciclovir.

17. The production process according to claim 13, wherein the target cell is a human corneal epithelial cell, a human corneal endothelial cell, a human conjunctival epithelial cell, or a human kidney-derived cell.

18. The production process according to claim 13, wherein human serum is used in the step (a) or (b).

19. A process for determining a candidate inducing factor for a target cell, comprising the steps of
( a' ) culturing a feeder cell according to claim 1 on a cell culture carrier,
(b' ) transducing a feeder cell according to claim 1 with the gene of the candidate inducing factor for the target cell, and culturing the obtained candidate inducing factor-transduced feeder cell on a cell culture carrier,
(c') culturing and growing the target cell by seeding the target cell on each of the cell culture carriers of the above (a') and (b') and then by allowing the target cell to stand, and
(d') evaluating the cells cultured on each of the cell culture carriers.
